# EUROPEAN PATENT APPLICATION

(11) **EP 4 837 914 A2**
(43) Date of publication of application: **11.11.2026**
(21) Application number: 26198918.0
(22) Date of filing: 03.02.2023
(51) Int. Cl.: C07F 5/00

(54) **METHODS FOR LARGE SCALE SYNTHESIS OF RADIONUCLIDE COMPLEXES**

(30) Priority: 04.02.2022 US 202263306822 P
(62) Document number(s) of the earlier application(s) in accordance with Art. 76 EPC: 23704451.6 / 4 472 678
(71) Applicant: Advanced Accelerator Applications, 92500 Rueil-Malmaison (FR)
(72) Inventor: BARBATO, Donato, 86077 Pozzilli (Isernia) (IT); BO, Michele, 86077 Pozzilli (Isernia) (IT); ROSSETTO, Mattia, 86077 Pozzilli (Isernia) (IT); TEDESCO, Mattia, 86077 Pozzilli (Isernia) (IT); VALERIO, Federico, 86077 Pozzilli (Isernia) (IT)
(74) Representative: Carpmaels & Ransford LLP

(57) **Abstract**

The present disclosure relates to methods of large scale synthesis of radionuclide complex solutions having a high activity for diagnostic and/or therapeutic purposes, their use in the commercial production of radioactive drug substances, and to respective solutions as well as containers comprising said solutions.

## Description

### Technical Field

The present disclosure relates to methods of large scale synthesis of radionuclide complex solutions having a high activity for diagnostic and/or therapeutic purposes, their use in the commercial production of radioactive drug substances, and to respective solutions as well as containers comprising said solutions.

### Background Art

The concept of targeted drug delivery is based on cell receptors or other cell surface markers which are overexpressed in the target cell in contrast to the not-to-be-targeted cells. If a drug has a binding site to those overexpressed cell surface markers, it allows the delivery of the drug after its systemic administration in high concentration to those target cells while leaving other cells, which are not of interest, unaffected. For example, if tumor cells are characterized by an overexpression of a specific cell receptor, a drug with binding affinity to said receptor will accumulate in high concentration in the tumor tissue after intravenous infusion while leaving the normal tissue unaffected.

This targeted drug delivery concept has also been used in nuclear medicine to selectively deliver radionuclides to target cells for diagnostic or therapeutic purposes. For this nuclear medicinal application, a target binding moiety is typically linked to a chelating moiety which is able to form a strong complex with the metal ions of a radionuclide. This radionuclide complex is then delivered to the target cell and the decay of the radionuclide is then releasing high energy electrons, positrons or alpha particles as well as gamma rays at the target site.

The radionuclide complex is preferably produced in a shielded closed-system due to significant radioactivity. In such shielded closed-system manufacturing, purification and formulation steps of the drug substance are part of a continuous process. Moreover, the decay of the radionuclide does not allow enough time for any interruption of the production process of the drug substance. Otherwise the desired activity of a drug product would not be attained putting diagnostic or therapeutic outcome at risk. Therefore, preferably no tests may be performed at critical steps and no synthesis intermediates may be isolated and controlled in the course of production.

Conventional industrial production methods of drug products such as those described in the WO2020/079799 A1 of the applicant comprising radionuclide complexes are disadvantageous in that solutions having higher activities of a radionuclide cannot be utilised as reactants, since they would lead to a considerable degree of radiolysis when mixed with a target binding molecule. This limits supply of certain radiodiagnostic and radiotherapeutic drug substances, while demand is increasing due to established medical progress in certain areas.

Thus, it is desirable to provide synthesis methods for the production of radionuclide complexes at high activities, wherein radiolysis is substantially avoided, so that per given unit of time a greater number of drug products (patient doses) having an activity that is suitable for administration to patients can be produced. A synthesis method for the production of radionuclide complexes as radioactive drug substance shall have the following advantages:
- a high labelling yield correlating with high radiochemical purity,
- a high labelling yield with minimized level of free (uncomplexed) radionuclide,
- a production of a larger number of doses per batch in comparison to conventional methods.

### Summary of the disclosure

The present disclosure relates to a reaction solution for radiolabeling a target binding organic molecule with ¹⁷⁷Lu(III) ions, wherein said reaction solution comprises:
(1) ¹⁷⁷Lu(III) ions in a volumetric activity of at least 17 GBq/mL,
(2) a target binding organic molecule comprising a target binding organic moiety linked to a chelating moiety suitable for chelating Lu(III) ions, and
(3) one or more stabilizers against radiolytic degradation.

The present disclosure also relates to a mother solution for preparing a dispensing solution comprising ¹⁷⁷Lu radiolabeled target binding organic molecule, wherein said mother solution comprises:
(1) ¹⁷⁷Lu(III) ions in a volumetric activity of at least 10 GBq/mL,
(2) a radionuclide complex formed by a target binding organic molecule comprising a target binding organic moiety linked to a chelating moiety and the ¹⁷⁷Lu(III) ions,
(3) one or more stabilizers against radiolytic degradation, and
(4) an oxygen concentration lower than 50 mg/L, preferably lower than 20 mg/L, more preferably lower than 10 mg/L, even more preferably lowever than 5 mg/L, even more preferably lower than 3 mg/L at 25 deg C.

The present disclosure also relates to a mother solution container for collecting solutions from a radiolabeling reaction, wherein said container comprises:
(1) a mother solution comprising:
   a. ¹⁷⁷Lu(III) ions in a volumetric activity of at least 10 GBq/mL,
   b. a radionuclide complex formed by a target binding organic molecule comprising a target binding organic moiety linked to a chelating moiety and the ¹⁷⁷Lu(III) ions,
   c. one or more stabilizers against radiolytic degradation, and
(2) a headspace gas volume above the mother solution, wherein said headspace gas volume contains not more than 10 vol% oxygen.

The present disclosure also relates to a process for manufacturing a radiopharmaceutical solution comprising the steps:
(1) providing a reaction solution,
(2) reacting a target binding organic molecule comprising a target binding organic moiety linked to a chelating moiety with ¹⁷⁷Lu(III) ions at below atmospheric pressure, optionally in the presence of an inert gas, to obtain a radionuclide complex in a single container for radiolabeling.

The present disclosure also relates to a product which is obtainable or obtained by the method as described herein. The present disclosure also relates to aqueous solutions comprising radionuclide complexes.

### Brief description of the drawings

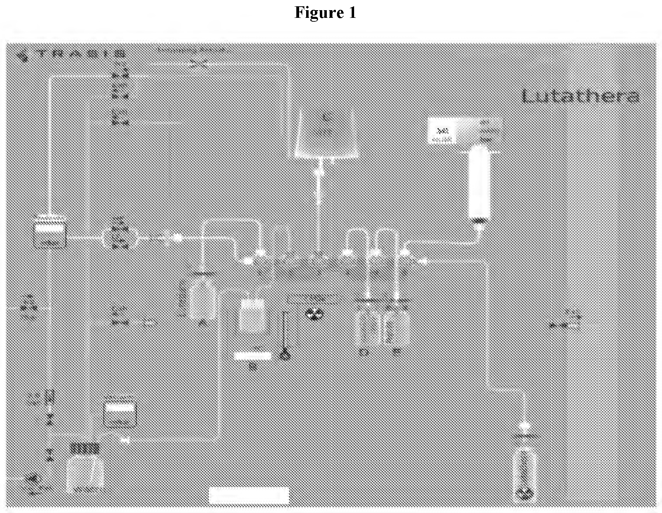

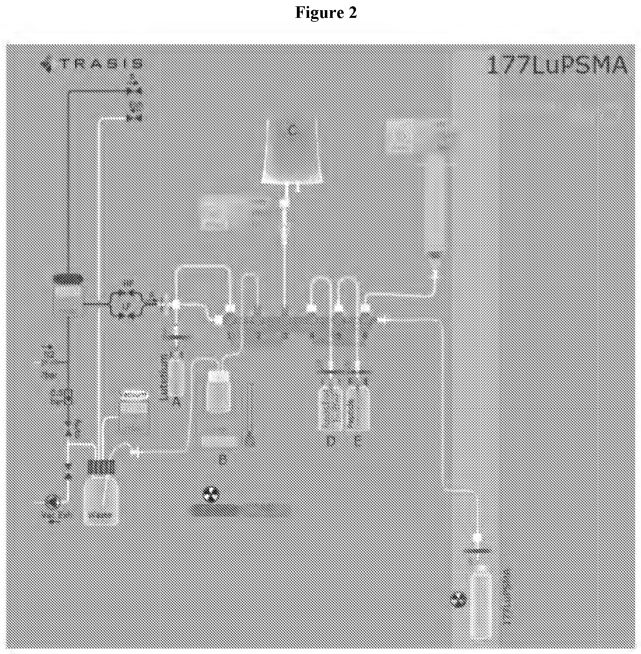

Figures 1 and 2 show the MiniAio Kit Cassette and its kit assembly (under Grade C) for 177Lu-DOTATATE and 177Lu-PSMA-617, respectively.

### Detailed Description

### Definitions

As used herein, the term "reaction solution" refers to a solution comprising ions of a radionuclide, a target binding organic molecule which is suitable for chelating the radionuclide ions, and one or more stabilizers against radiolytic degradation. The target binding organic molecule comprises a target binding organic moiety linked directly or indirectly to a chelating moiety.

As used herein, the term "mother solution" refers to a solution which is obtained when the aforesaid reaction solution has finished reacting forming radionuclide complexes, has been processed as described further below (if applicable) and has been mixed and diluted with water for injection (WFI) (if applicable).

As used herein, the term "dispensing solution" refers to a solution which is obtained when the aforesaid mother solution has been additionally mixed with a dilution solution. The dispensing solution comprises all the components and an activity that is suitable for patient administration. The dispensing solution is the solution that is dispensed into multiple patient doses (vials), which are destined for subsequent administration to a patient without further material change.

The methods of the present disclosure are particularly adapted for use of radionuclides of metallic nature and which are useful in medicine for diagnostic and/or therapeutic purposes. Such radionuclide includes, without limitation, the radioactive isotopes of I, In, Tc, Ga, Cu, Zr, Pb, Bi, Ac, Th, Re, Sc, Tb, Y and Lu, and in particular: ¹³¹I, ¹¹¹In, ^{99m}Tc, ⁶⁸Ga, ⁶⁴Cu, ⁶⁷Cu, ⁸⁹Zr, ²¹²Pb, ²¹³Bi, ²²⁵Ac, ²²⁷Th, ⁴⁷Sc, ¹⁸⁸Re, ¹⁶¹Tb, ⁹⁰Y, ¹⁷⁷Lu. The ions of the radioisotopes form non-covalent bonds with the functional groups of the chelating agent, e.g. amino groups or carboxyl groups.

In a preferred embodiment, the radionuclide ions comprise lutetium-177 (¹⁷⁷Lu) ions. For example, the radionuclide ions may originate from ¹⁷⁷LuCl₃ in HCl solution.

As used herein, the term "stabilizer against radiolytic degradation" refers to a stabilizing agent which protects organic molecules against radiolytic degradation, e.g. when a gamma ray emitted from the radionuclide is cleaving a bond between the atoms of an organic molecules and radicals are forms, those radicals are then scavenged by the stabilizer which avoids the radicals undergo any other chemical reactions which might lead to undesired, potentially ineffective or even toxic molecules. Therefore, those stabilizers are also referred to as "free radical scavengers" or in short "radical scavengers". Other alternative terms for those stabilizers are "radiation stability enhancers", "radiolytic stabilizers", or simply "quenchers".

Stabilizer(s) present in the solutions of the present disclosure may be selected from gentisic acid (2,5-dihydroxybenzoic acid) or salts thereof, ascorbic acid (L-ascorbic acid, vitamin C) or salts thereof (e.g. sodium ascorbate), methionine, histidine, melatonine, ethanol, and Se-methionine, preferably selected from gentisic acid or salts thereof, preferably not ethanol.

In specific embodiments, the reaction solution and mother solution do not include ascorbic acid, preferably they include gentisic acid as stabilizer agent but not ascorbic acid. In specific embodiments, the reaction solution and mother solution do not include ethanol as stabilizing agent. Preferably the reaction solution and mother solution do not include either of ascorbic acid and ethanol as stabilizers.

As used herein, when a numerical value or range is preceded by "about", the "about" indicates a deviation of the value or range by ± 20%, preferably ± 10%, more preferably ± 5%, potentially ± 2% or ± 1%.

Lutetium-177 is accessible via (n,y) reaction. There are two methods of ¹⁷⁷Lu production in a nuclear reactor. One method comprises irradiation of ¹⁷⁶Lu, leading to the direct formation of ¹⁷⁷Lu. However, this method leads to concomitant formation of the metastable ^{177m}Lu isotope and other lutetium isotopes. Due to difficulties and challenges of separating the isotopes a composition comprising ¹⁷⁷Lu and ^{177m}Lu and others may be used. Such a composition comprising ¹⁷⁷Lu and related isotopes is called carrier-added ¹⁷⁷Lu source or ¹⁷⁷Lu (C.A.) source.

The second method involves beta decay of the short-lived radioisotope ¹⁷⁷Yb (half-life of 1.9 hours), which is produced by neutron capture of enriched ¹⁷⁶Yb (> 99%) target. The low thermal neutron cross section of the ¹⁷⁶Yb (n,y) ¹⁷⁷Yb reaction (2.1 barn), however, results in a production of only very small amounts of the desired ¹⁷⁷Lu in comparison with the total mass of the target. However, a tedious separation of ¹⁷⁷Lu from ¹⁷⁶Yb is feasible leading to a composition comprising only the ¹⁷⁷Lu isotope. Such compositions provide non-carrier-added ¹⁷⁷Lu, in short ¹⁷⁷Lu (N.C.A.).

### The target binding molecule for use according to the disclosure

The target binding molecule as used herein comprises (i) a targeting binding organic moiety, linked to (ii) a chelating moiety, either directly or indirectly via a linker.

As used herein, the term "target binding organic moiety" refers to an organic moiety which has specific binding affinity to a target protein, typically a cell surface receptor or cellular protein. In specific embodiments, said target binding receptor moiety is an organic moiety which has specific binding affinity to somatostatin receptor, for example at least somatostatin receptor subtype 2 (SSTR2) or an organic moiety which has binding affinity to prostate specific membrane antigen (PSMA). Other targets/target binding organic moieties may be Gastrin-Releasing Peptide Receptor (GRPR) antagonists, ligands targeting αvβ3/αvβ5 integrins, fibroblast activation protein (FAP) inhibitors.

As used herein, the term "chelating moiety" refers to an organic moiety comprising functional groups that form non-covalent bonds with a radionuclide during the reacting step of the method and, thereby, form a stable radionuclide complex. The chelating moiety in the context of the present invention may be or may comprise 1,4,7,10-Tetraazacyclododecane-1,4,7,10-tetraacetic acid (DOTA), 1,4,7,10-Tetraazacyclododecane-1-(glutaric acid)- 4,7,10 -triacetic acid (DOTAGA), diethylentriaminepentaacetic acid (DTPA), nitrilotriacetic acid (NTA), ethylenediaminetetraacetic acid (EDTA), 1,4,7,10-tetraazacyclododecane-1,4,7-triacetic acid (DO3A), 1,4,7-triazacyclononane-1,4,7-triacetic acid (NOTA), 1-(1,3-carboxypropyl)-4,7-carboxymethyl-1,4,7-triazacyclononane (NODAGA) or mixtures or variants thereof, preferably DOTA.

Such chelating moiety is either directly linked to the target binding organic moiety or connected via a linker molecule, preferably it is directly linked. The linking bond(s) is (are) either covalent or non-covalent bond(s) between the target binding organic moiety (and the linker) and the chelating moiety, preferably the bond(s) is (are) covalent.

### SSTR binding molecule

In specific embodiments, said target binding organic molecule comprises somatostatin receptor binding peptides. As used herein, the term "somatostatin receptor binding peptide" refers to a peptidic moiety with specific binding affinity to the somatostatin receptor for example at least somatostatin receptor subtype 2 (SSTR2).

In specific embodiments, said target-binding molecule for use as described herein is a compound of formula

C-S-P

wherein :
- C is a chelating agent capable of chelating a radionuclide;
- S is an optional spacer covalently linked between C and P;
- P is a somatostatin receptor binding peptide covalently linked to C, for example via its N-terminal end, either directly or indirectly via S.

Such somatostatin receptor binding peptide may be selected from octreotide, octreotate, lanreotide, vapreotide, and pasireotide, preferably selected from octreotide and octreotate.

As used herein, the term "somatostatin receptor binding peptide" refers to a peptidic moiety with specific binding affinity to somatostatin receptor. Such somatostatin receptor binding peptide may be selected from octreotide, octreotate, lanreotide, vapreotide, and pasireotide, preferably selected from octreotide and octreotate.

The somatostatin receptor-binding peptide linked to a chelating moiety may comprise a chelating moiety selected from the group comprising DOTA, DOTAGA, DTPA, NTA, EDTA, DO3A, NOTA, NODAGA. The somatostatin receptor-binding peptide linked to a chelating moiety comprises preferably DOTA.

According to preferred embodiments the target binding organic moiety linked to the chelating moiety wherein the target binding organic moiety is a somatostatin receptor-binding peptide may be selected from DOTA-OC, DOTA-TOC (edotreotide), DOTA-NOC, DOTA-TATE (oxodotreotide), DOTA-LAN, and DOTA-VAP, preferably selected from DOTA-TOC and DOTA-TATE, more preferably DOTA-TATE.

Accordingly, the cell receptor binding moiety and the chelating agent may form together the following molecules:
DOTA-OC: [DOTA⁰,D-Phe¹]octreotide,
DOTA-TOC: [DOTA⁰,D-Phe¹,Tyr³]octreotide, edotreotide (INN),
represented by the following formulas: 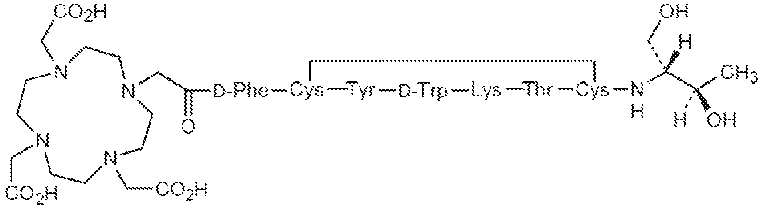 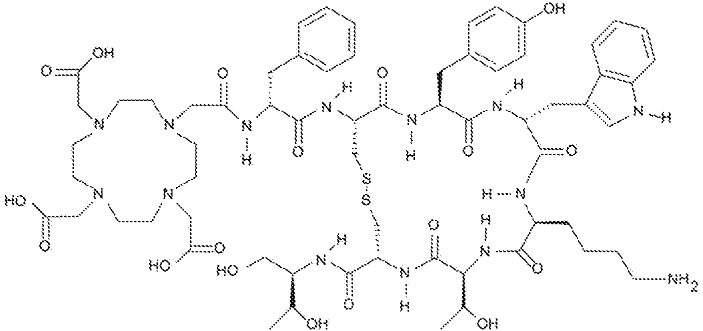
DOTA-NOC: [DOTA⁰, D-Phe¹,1-Nal³]octreotide,
DOTA-TATE: [DOTA⁰,D-Phe¹,Tyr³]octreotate, DOTA-Tyr³-Octreotate, DOTA-d-Phe-Cys-Tyr-d-Trp-Lys-Thr-Cys-Thr (cyclo 2,7), oxodotreotide (INN), represented by the following formula : 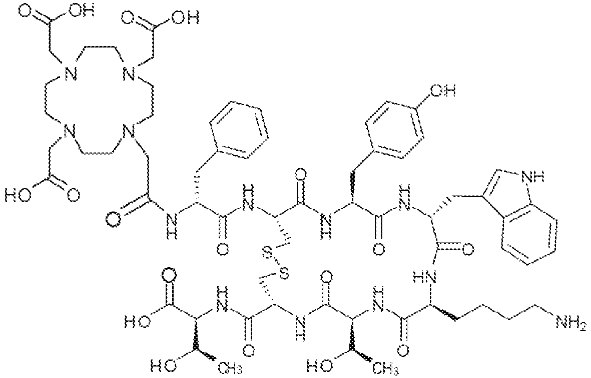
DOTA-LAN: [DOTA⁰,D-β-Nal¹]lanreotide,
DOTA-VAP: [DOTA⁰,D-Phe¹,Tyr³]vapreotide.

### Satoreotide trizoxetan

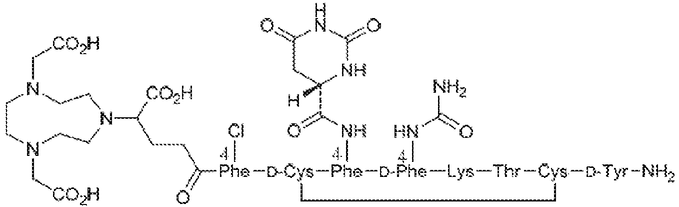

### Satoreotide tetraxetan

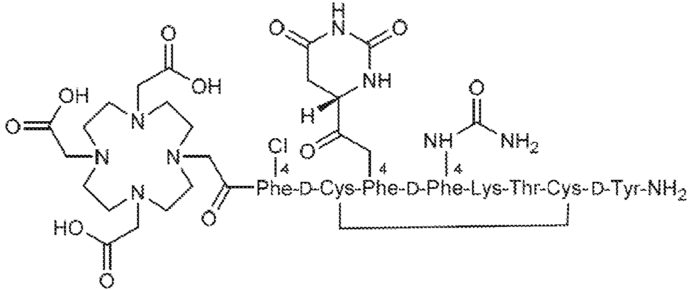

### PSMA-binding molecule

In specific embodiments, said target binding organic molecule comprises a PSMA-binding moiety. The PSMA-binding moiety may comprise one or more glutamate-urea-lysine moieties.

The PSMA-binding organic molecule may comprise a chelating moiety selected from the group comprising DOTA, DOTAGA, DTPA, NTA, EDTA, DO3A, NOTA, NODAGA, preferably DOTA or DOTAGA, more preferably DOTA.

According to preferred embodiments the target binding organic molecule is a PSMA-binding peptide preferably selected from PSMA-617, wherein PSMA-617 has the structure 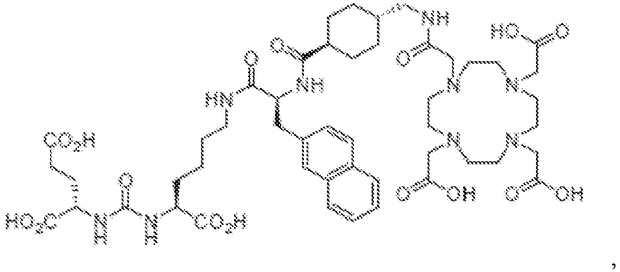 or variants of PSMA-617 with additional structural features like albumin-binding moieties; PSMA-I&T, wherein PSMA-I&T has the structure 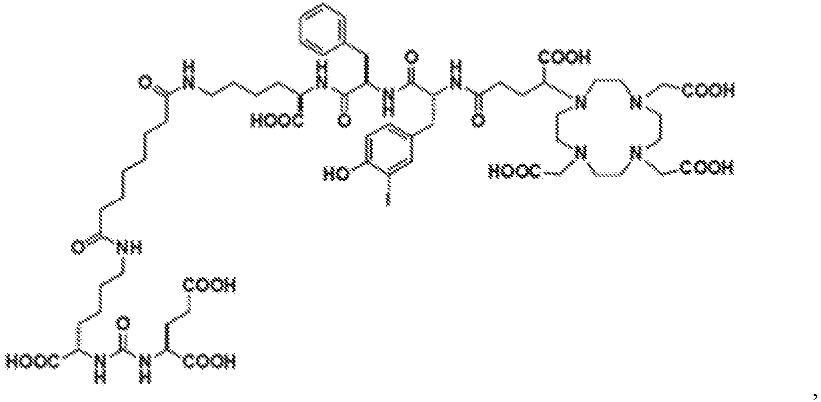 and PSMA-R2, wherein PSMA-R2 has the structure 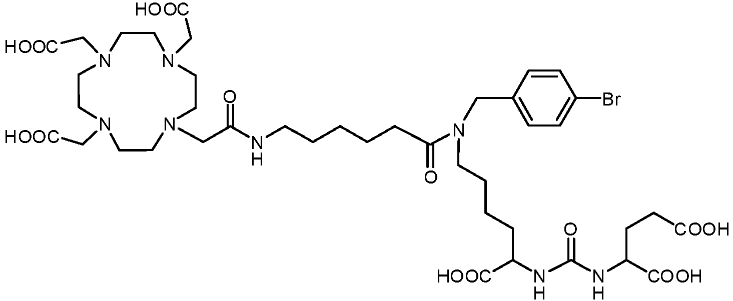 with the Glu and Lys residue being in the L-configuration, with PSMA-617 being preferred.

PSMA ligand or PSMA-binding organic molecule according to the present disclosure many be selected from the group consisting of PSMA-617, PSMA I&T, PSMA-R2, MIP-1095, MIP-1545, MIP, MIP-1555, MIP-1557, MIP-1558, CTT1403, FC705, BAY-2315497, TLX592, PSMA-TCC, rhPSMA, rhPSMA-7, rhPSMA-7.3, PSMA-7 I&T, EB-PSMA-617, PSMA-ALB-02, PSMA-ALB-053, PSMA-ALB-056, P16-093, PSMA-93, and RPS-074, preferably selected from the group consisting of PSMA-617, PSMA I&T, and PSMA-R2, more preferably PSMA-617.

### The reaction solution

The present disclosure concerns a reaction solution which comprises reactants which are necessary to form a radionuclide complex by way of a reaction. Such reactants are a radionuclide and a target binding organic molecule. The reaction of forming a radionuclide complex from said two reactants is also called radiolabelling. Thus, the reaction solution is used for and is suitable for radiolabelling a target binding organic molecule with a radionuclide. The target binding organic molecule comprises (i) a targeting binding organic moiety, linked to (ii) a chelating moiety, either directly or indirectly via a linker, as described above in the preceding section.

The reaction solution additionally comprises one or more stabilizers against radiolytic degradation.

The radionuclide may preferably be lutetium-177 (¹⁷⁷Lu) in the form of ¹⁷⁷Lu(III) ions. For example, the radionuclide ions may originate from ¹⁷⁷LuCl₃ in HCl solution. The reaction solution may comprise the ¹⁷⁷Lu(III) ions in a volumetric activity of at least 17 GBq/ml, or 18 GB/q/ ml, preferably at least 19 GBq/ml and more preferably at least 20 GBq/mL, even more preferably at least 25 GBq/mL, even more preferably at least 28 GBq/mL, even more preferably at least 30 GBq/mL. Upper limits in relating to the before mentioned minimum values may be 20, 25, 30, 40, 50 GBq/mL.

The present disclosure is related to a reaction solution for radiolabeling a target binding organic molecule with ¹⁷⁷Lu(III) ions, wherein said reaction solution comprises:
(1) ¹⁷⁷Lu(III) ions in a volumetric activity of at least 17 GBq/mL,
(2) a target binding organic molecule comprising a target binding organic moiety linked to a chelating moiety suitable for chelating Lu(III) ions, and
(3) one or more stabilizers against radiolytic degradation.

The reaction solution may comprise an oxygen concentration lower than 50 mg/L, preferably lower than 20 mg/L, more preferably lower than 10 mg/L, even more preferably lowever than 5 mg/L, even more preferably lower than 3 mg/L at 25 deg C or an oxygen concentration which is lower than 7 mg/L or 6 mg/L, or 5 mg/L, preferably lower than 4 mg/L, more preferably lower than 3 mg/L, more preferably lower than 2 mg/L or 1 mg/L (all values at 25 degrees Celsius). Oxygen may be substantially absent in the reaction solution. A low oxygen concentration in the reaction solution reduces radiolytic degradation. The low levels/absence of oxygen may be achieved by degassing and/or flushing the reaction solution with a protection gas, such as nitrogen or argon.

Due to a low oxygen concentration ¹⁷⁷Lu(III) ions may be comprised in the reaction solution in a volumetric activity of at least 17 GBq/ml, or 18 GBq/ml, preferably 19 GBq/ml and more preferably 20 GBq/ml, even more preferably at least 28 GBq/mL, even more preferably at least 30 GBq/mL. Upper limits in relating to the before mentioned minimum values may be 20, 25, 30, 40, 50 GBq/mL.

The reaction solution contains one or more stabilizers against radiolytic degradation and these may comprise gentisic acid or a salt thereof. The concentration of gentisic acid or a salt thereof may be from 5 to 15 mg/mL.

In case the target binding organic moiety linked to a chelating moiety is a somatostatin receptor-binding peptide, the concentration of gentisic acid or salt thereof may be 10 to 15 mg/mL.

In case the target binding organic moiety linked to a chelating moiety is a PSMA-binding peptide, the concentration of gentisic acid or salt thereof is from 5 to 10 mg/mL.

The reaction solution may comprise ascorbic acid or a salt thereof in not more than 5% (w/w), preferably not more than 2%, even more preferably not more than 1%. Most preferably, the reaction solution does not comprise ascorbic acid, i.e. it is substantially absent (substantially 0%).

The reaction solution may comprise ethanol in not more than 5%(w/w), preferably not more than 2%, even more preferably not more than 1%. Most preferably, the reaction solution does not comprise ethanol, i.e. it is substantially absent (substantially 0%).

In preferred embodiments, ascorbic acid or salts thereof and ethanol are substantially not included in the reaction solution.

The reaction solution may comprise the target binding organic molecule in molar excess to the ¹⁷⁷Lu(III) ions, even when the ¹⁷⁷Lu(III) ions originate from a non-carrier-added (N.C.A.) ¹⁷⁷Lu(III) ion source. The molar ratio between the target binding organic molecule and the ¹⁷⁷Lu(III) ions may be at least 1.2, preferably between 1.5 and 3.5.

The reaction solution may comprise the target binding organic molecule in molar excess to the group of all Lu(III) ions including ¹⁷⁷Lu(III) ions, ¹⁷⁶Lu(III) ions, ¹⁷⁵Lu(III), and metastable ^{177m}Lu(III) ions, which are present in the composition that provides the ¹⁷⁷Lu(III) ions at the volumetric activity given above for use in the instant reaction solution, when carrier-added (C.A.) ¹⁷⁷Lu(III) is used as a source of ¹⁷⁷Lu(III) ions. The molar ratio between the target binding organic molecule and the group of all Lu(III) ions including ¹⁷⁷Lu(III) ions, ¹⁷⁶Lu(III) ions, ¹⁷⁵Lu(III), and metastable ^{177m}Lu(III) ions may be at least 1.2, preferably between 1.5 and 3.5.

The reaction solution may comprise a pharmaceutically acceptable buffer to provide a pH in the range of 2 to 8, which is suitable for the reaction between the ¹⁷⁷Lu(III) ions and the target binding organic molecule. The pharmaceutically acceptable buffer preferably provides a pH in the range of 4 to 6.

The pharmaceutically acceptable buffer comprises acetate buffer, citrate buffer or a phosphate buffer. The citrate buffer may comprise a citrate and HCl and/or citric acid. The phosphate buffer may comprise sodium dihydrogen phosphate and disodium hydrogen phosphate. The pharmaceutically acceptable buffer comprises preferably an acetate buffer, which is preferably composed of acetic acid and sodium acetate.

### The mother solution

The present disclosure concerns a mother solution which is a solution obtained when the reaction solution described in the preceding section has finished reacting forming radionuclide complexes, has been processed as described further below and has been mixed and diluted with water for injection (WFI). As the radiolabelling reaction is terminated, the mother solution comprises a radiolabelled target binding organic molecule, which is a radionuclide complex formed by the target binding organic moiety linked directly or indirectly to a chelating moiety and the 177Lu(III) ions described above.

The mother solution is used for and is suitable for subsequently preparing a dispensing solution. The dispensing solution is a solution that is dispensed into multiple patient doses (vials), which are destined for subsequent administration to a patient without further material change.

The present disclosure concerns a mother solution for preparing a dispensing solution comprising 177Lu radiolabeled target binding organic molecule, wherein said mother solution comprises:
(1) ¹⁷⁷Lu(III) ions in a volumetric activity of at least 10 GBq/mL,
(2) a radionuclide complex formed by a target binding organic molecule comprising target binding organic moiety linked to a chelating moiety and the 177Lu(III) ions,
(3) one or more stabilizers against radiolytic degradation, and
(4) preferably, an oxygen concentration lower than 50 mg/L, preferably lower than 20 mg/L, more preferably lower than 10 mg/L, even more preferably lower than 5 mg/L, even more preferably lower than 3 mg/L at 25 deg C or an oxygen concentration lower than 7 mg/L, preferably lower than 5 mg/L, more preferably lower than 4 mg/L, even more preferably lower than 3 mg/L, even more preferably lower than 2, even more preferably lower than 1 mg/L (all values at 25 deg C), even more preferably the mother solution is substantially free of oxygen.

The mother solution may comprise the ¹⁷⁷Lu(III) ions in a volumetric activity of at least 10 GBq/ml, at least 11 GBq/ml, at least 12 GBq/ml, preferably at least 13 GBq/ml, more preferably at least 15 GBq/ml most preferably at least 16 GBq/ml.

The radionuclide complex formed by a target binding organic molecule and the 177Lu(III) ions comprises the target binding organic molecule as described above under the section "target binding molecule for use according to the disclosure".

The one or more stabilizers against radiolytic degradation and their specifics are those as described above with respect to the reaction solution. A stabilizer may be gentisic acid or its salts and in concentration ranges as described above with respect to the reaction solution.

The oxygen concentration and its specifics are those as described above with respect to the reaction solution.

The mother solution may additionally comprise a nitrogen concentration of up to 20 ml/L at 25 degrees Celsius or an argon concentration of up to 60ml/L at 25 degrees Celsius. The mother solution may comprise a nitrogen concentration in a range of 3 to 20 ml/L 25 degrees Celsius, preferably 5 to 15, more preferably 10 to 15 ml/L at 25 degrees Celsius. Alternatively, the mother solution may additionally comprise a nitrogen concentration of up to 20 mg/L at 25 degrees Celsius or an argon concentration of up to 60 mg/L at 25 degrees Celsius. Alternatively, the mother solution may comprise a nitrogen concentration in a range of 3 to 20 mg/L 25 degrees Celsius, preferably 5 to 15, more preferably 10 to 15 mg/L at 25 degrees Celsius. Alternatively, the mother solution may comprise an argon concentration of 3 to 60 mg/L at 25 degrees Celsius, preferably 10 to 50, more preferably 20 to 40 mg/L at 25 degrees Celsius.

The presence of an inert gas like nitrogen or argon in the mother solution is a consequence of the steps leading up to the formation of the mother solution as part of the process described further below. The concentration of inert gas in the mother solution reduces radiolytic degradation of the components of the mother solution.

Methods for determining oxygen, nitrogen and argon concentrations and content in aqueous solutions or in the gas phase are well known and have been described manifold in the literature and encyclopedias , e.g. Determination of Argon in Air and Water, J. Lasa et al., Chem. Anal. (Warsaw), 47, 839 (2002), H. H. Willard et al., Instrumental methods of analysis, 6th ed. D. Van Norstrand, New York, 1981, pages 910-912; M. L. Hitchman, Measurement of dissolved oxygen, John Wiley & Sons, New York 1978; Ullmann's Encyclopedia of Industrial Chemistry; S. Uchiyama, Analysis of Dissolved Argon, Oxygen, and Nitrogen in Solutions, Shimadzu Corporation publication, July 2021.

The description of ascorbic acid or salts thereof and ethanol; target binding organic molecule; the molar excess of the target binding organic molecule over Lu(III) ions; and the pH and pharmaceutically acceptable buffer given above under the reaction solution apply equally to the mother solution.

In specific embodiments, the mother solution may comprise as preferred radionuclide complexes ¹⁷⁷Lu-DOTA-TOC (Lutetium (¹⁷⁷Lu) edotreotide) or ¹⁷⁷Lu-DOTA-TATE (Lutetium (¹⁷⁷Lu) oxodotreotide) or ¹⁷⁷Lu-PSMA-617 ([¹⁷⁷Lu]Lu-PSMA-617, Lutetium (¹⁷⁷Lu) vipivotide tetraxetan [INN] or Lutetium Lu 177 vipivotide teraxetan [USAN]) or ¹⁷⁷Lu-PSMA-I&T (Lutetium (¹⁷⁷Lu) zadavotide guraxetan).

Throughout the entire present disclosure, the radionuclide complex ¹⁷⁷Lu-PSMA-617 (Lutetium (¹⁷⁷Lu) vipivotide tetraxetan) may be also referred to as [¹⁷⁷Lu]Lu-PSMA-617, Lutetium (¹⁷⁷Lu) vipivotide tetraxetan [INN] or Lutetium Lu 177 vipivotide teraxetan [USAN], PLUVICTO, or 2-[4-[2-[[4-[[(2S)-1-[[(5S)-5-carboxy-5-[[(1S)-1,3-dicarboxy-propyl]carbamoylamino]pentyl]amino]-3-naphthalen-2-yl-1-oxopropan-2-yl] carbamoyl] cyclohexyl]methylamino]-2-oxoethyl]-4,7,10-tris(carboxylatomethyl)-1,4,7,10-tetraza cyclododec-1-yl]acetate; lutetium-177(3+). The molecular mass is 1216.06 g/mol and the molecular formula is C₄₉H₆₈¹⁷⁷LuN₉O₁₆. The chemical structure for lutetium Lu 177 vipivotide tetraxetan is shown below:

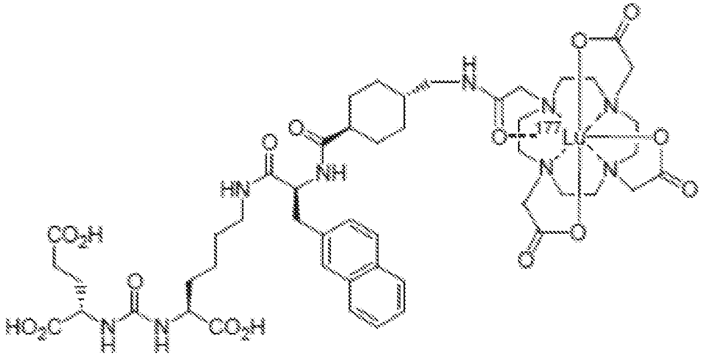

The mother solution may comprise the radionuclide complexes ¹⁷⁷Lu-DOTA-TOC (¹⁷⁷Lu-edotreotide) or ¹⁷⁷Lu-DOTA-TATE (¹⁷⁷Lu-oxodotreotide), preferably ¹⁷⁷Lu-DOTA-TATE (¹⁷⁷Lu-oxodotreotide), in a volumetric activity of 12 GBq/ml to 17 GBq/ml.

In other specific embodiments, the mother solution may comprise the radionuclide complex ¹⁷⁷Lu PSMA-617 in a volumetric activity of 10 to 30, preferably 10 to 25, more preferably 15 to 25, even more preferably 17 to 25, even more preferably 17 to 20, even more preferably 18 to 19 GBq/ml.

The mother solution may comprise the radionuclide complex ¹⁷⁷Lu-PSMA I&T in a volumetric activity of 10 to 30, preferably 10 to 25, more preferably 15 to 25, even more preferably 17 to 25, even more preferably 17 to 20, even more preferably 18 to 19 GBq/ml.

### The mother solution container

The present disclosure concerns a mother solution container, which is used for collecting solutions formed in a radiolabelling reaction, preferably after completion of a radiolabelling reaction. The mother solution container comprises the mother solution described in the preceding section. The mother solution container also comprises a headspace gas volume above the mother solution.

The present disclosure relates to a mother solution container for collecting solutions formed in a radiolabeling reaction, wherein said container comprises:
(1) a mother solution comprising:
   a. ¹⁷⁷Lu(III) ions in a volumetric activity of at least 10 GBq/mL,
   b. a radionuclide complex formed by a target binding organic molecule comprising a target binding organic moiety linked to a chelating moiety and the ¹⁷⁷Lu(III) ions,
   c. one or more stabilizers against radiolytic degradation, and
(2) a headspace gas volume above the mother solution, wherein said headspace gas volume contains not more than 10 vol% oxygen.

The mother solution container comprises a headspace gas volume above the mother solution, wherein said headspace gas volume contains not more than 10 vol%, preferably not more than 7 vol%, more preferably not more than 5 vol%, most preferably not more than 3 vol% oxygen. The headspace gas volume may substantially contain no oxygen (substantially 0 vol%). A low volume percentage of oxygen in the headspace gas volume reduces radiolytic degradation of the components of the mother solution.

Since the mother solution container comprises the mother solution described in the preceding section, all features and embodiments described above under the mother solution apply equally to the mother solution container.

In an embodiment, to the mother solution container is added a dilution solution, wherein the dilution solution comprises a stabilizer against radiolytic degradation, a sequestering agent, and optionally an isotonic agent. In such embodiment, the mother solution container additionally comprises:
(3) a stabilizer against radiolytic degradation,
(4) a sequestering agent, and
(5) optionally an isotonic agent.

The stabilizer against radiolytic degradation may be selected among any stabilized mentioned herein above, preferably ascorbic acid or salts thereof. Ethanol may be present in concentrations described above under the reaction solution, but is preferably substantially not contained in the dilution solution.

As used herein, "sequestering agent" refers to a chelating agent suitable to complex free radionuclide metal ions in the formulation (which are not complexed with the radiolabelled peptide). The sequestering agent is preferably di-ethylene-triamine-penta-acetic acid (DTPA, also called pentetic acid).

The optional isotonic agent may be any selected from monosodium or disodium phosphate, sodium, potassium, calcium or magnesium chloride and the like or mixtures of such salts, preferably sodium chloride.

In a preferred embodiment, the mother solution container additionally comprises:
(3) a stabilizer against radiolytic degradation, preferably ascorbic acid or salts thereof,
(4) a sequestering agent, preferably DTPA, and
(5) optionally an isotonic agent, preferably NaCl.

In an embodiment, the mother solution container comprises the necessary components to constitute a dispensing solution which is suitable for dispensing into multiple patient doses (vials), which are destined for subsequent administration to a patient without further material change.

In such an embodiment the mother solution container comprises:
(A) ¹⁷⁷Lu-DOTATATE in an activity in the range from 296 to 444 MBq/mL, preferably 333 to 407 MBq/ml, more preferably about 351 to 389 MBq/ml, most preferably about 370 MBq/mL (10 mCi/mL);
(B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
(C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
(D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
(E) ascorbic acid or a salt thereof in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
(F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
(G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
(H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.

In such other embodiment the mother solution container comprises:
(A) ¹⁷⁷Lu-PSMA-617 in an activity in the range from 800 to 1200 MGq/ml, preferably 900 to 1100 MBq/ml, more preferably 950 to 1050 MBq/ml, most preferably about 1000 MBq/mL (27 mCi/mL);
(B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
(C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
(D) gentisic acid or a salt thereof in a concentration in the range from 0.3 to 1.0 mg/mL, preferably 0.3 to 0.5 mg/mL, more preferably 0.31 to 0.47 mg/mL, even more preferably 0.35 to 0.43 mg/mL, even more preferably 0.37 to 0.41, most preferably about 0.39 mg/mL with regard to the free acid;
(E) ascorbic acid or a salt thereof in a concentration in the range from 20 to 52.5 mg/mL, preferably 47.5 to 52.5 mg/mL, more preferably 48 to 52 mg/mL, most preferably about 50 mg/mL with regard to the sodium salt;
(F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

### The process for manufacturing a radiopharmaceutical solution

The present disclosure also relates to a process for manufacturing a radiopharmaceutical solution, which affords the solutions and the container, respectively, presenting the features described in the preceding sections.

The process for manufacturing a radiopharmaceutical solution comprises the steps:
(1) providing a reaction solution,
(2) reacting a target binding organic molecule comprising a target binding organic moiety linked to a chelating moiety with ¹⁷⁷Lu(III) ions at below atmospheric pressure, optionally in the presence of an inert gas, to obtain a radionuclide complex in a single container for radiolabeling.

Step (1) concerns the reaction solution as described above under the section reaction solution, so that all features and embodiments described above apply equally to the reaction solution referred to in this section.

Step (1) of providing the reaction solution may comprise mixing the individual components described above. In particular, the stabilizer against radiolytic degradation may be mixed with the ¹⁷⁷Lu(III) ions prior to its mixing with the target binding organic moiety linked to a chelating moiety. Said individual components may be mixed under ambient atmosphere and ambient pressure to form the reaction solution.

Step (1) may alternatively comprise mixing the individual components described herein at below atmospheric pressure to form the reaction solution. Below atmospheric pressure comprises a pressure range that is suitable to remove gaseous components from a container up to removing gaseous components from a solution, but a pressure that would lead to significant evaporation of the solvent (water) is to be avoided. The pressure may be at least 150 mbar, 200 mbar, 250 mbar or 300 mbar below atmospheric pressure up to 400 mbar, 500 mbar, 650 mbar or 700 mbar below atmospheric pressure. The pressure may be preferably at least around 250 mbar and up to 500 mbar below atmospheric pressure.

Step (1) may comprise degassing solutions of the individual components described herein by letting an inert gas bubble through the solutions or purging the headspace above the individual solutions by an inert gas and then mixing the individual solutions under an inert gas atmosphere.

Step (1) may comprise degassing solutions of the individual components described herein by letting an inert gas bubble through the solutions or purging the headspace above the individual solutions by an inert gas and then mixing the individual solutions at below atmospheric pressure to form the reaction solution. Below atmospheric pressure may comprise a pressure as described above.

Mixing at below atmospheric pressure and/or degassing reduces the concentration of oxygen in the reaction solution, thereby reducing radiolytic degradation.

Step (1) may comprise providing the reaction solution in a container. This is preferably one single container. Providing the reaction solution in a container may comprise applying a pressure below atmospheric pressure as described above prior to the above step of mixing the individual components in the single container.

In step (1), the reaction solution may have an activity of at least 5 Ci, preferably, of from 5 to 20 Ci, more preferably of 5-15 Ci, even more preferably of 5 - 12, even more preferably of from about 5.4 to 12 Ci, even more preferably from 7 to 12, even more preferably from about 8 to 12 Ci.

In step (2) the target binding organic molecule and the ¹⁷⁷Lu(III) ions, which are comprised in the reaction solution, are reacted with each other at below atmospheric pressure to obtain a radionuclide complex composed of the target binding organic molecule and the ¹⁷⁷Lu(III) ions in a single container for radiolabeling. Below atmospheric pressure comprises applying a pressure as described above under step (1). Carrying out the reaction at below atmospheric pressure reduces radiolytic degradation. Step (2) comprises carrying out the reaction in one single container.

In step (2) the single container for radiolabelling comprises an oxygen concentration lower than 7 mg/L or 6 mg/L, or 5 mg/L, preferably lower than 4 mg/L, more preferably lower than 3 mg/L, more preferably lower than 2 mg/L or 1 mg/L (all values at 25 degrees Celsius). Oxygen may be substantially absent in the single container. A low oxygen concentration in the single container reduces radiolytic degradation. Due to a low oxygen concentration ¹⁷⁷Lu(III) ions may be comprised in the single container in a volumetric activity of at least 17 GBq/ml, or at least 18 GB/q/ ml, at least preferably at least 19 GBq/ml and more preferably at least 20 GBq/ml, even more preferably at least 25 GBq/mL, even more preferably at least 30 GBq/mL. Upper limits in relating to the before mentioned minimum values may be 20, 25, 30, 40, 50 GBq/mL.

In step (2) a molar excess of the target binding organic molecule over the ¹⁷⁷Lu(III) ions as described above is reacted, to ensure high radiochemical labelling yields. Advantageously, in certain preferred embodiments the process does not comprise any purification steps to remove free (non-chelated) ¹⁷⁷Lu(III) ions, such as a tC18 solid phase extraction (SPE) purification step. The use of a tC18 cartridge to perform a solid phase extraction (SPE) purification step to remove free (non-chelated) ¹⁷⁷Lu(III) ions presents some disadvantages. In particular, the use of this cartridge may require the elution of the product with ethanol, which is undesired (A. Mathur et al., Cancer Biother. Radiopharm. 2017, 32, 266-273). The use of a tC18 cartridge may also remove the stabilizers, which then need to be added again (S. Maus et al., Int. J. Diagnostic imaging, 2014, 1, 5-12).

The process may comprise a step (3) of recovering the radionuclide complex, which is formed in step (2) to obtain a mother solution. Step (3) concerns the mother solution as described above under the section mother solution, so that all features and embodiments described above apply equally to the mother solution referred to in this section to the extent they are applicable.

Step (3) may comprise recovering the radionuclide complex at below atmospheric pressure, wherein the pressure range is as given above. Step (3) may comprise recovering the radionuclide complex under an inert gas atmosphere. Preferably, in step (3) recovering the radionuclide complex is achieved under an inert gas atmosphere. The inert gas atmosphere may be provided by nitrogen or argon.

Step (3) of recovering the radionuclide complex may comprise introducing or transferring the mother solution into a single container affording a mother solution container. Step (3) concerns the mother solution container as described above under the section mother solution container, so that all features and embodiments described above apply equally to the mother solution container referred to in this section to the extent they are applicable.

Step (3) of recovering the radionuclide complex may comprise purging the mother solution container with an inert gas before and during introducing or transferring the mother solution into the mother solution container.

Step (3) of recovering the radionuclide complex may comprise purging the mother solution container with an inert gas at a pressure of at least 250 mbar above atmospheric pressure before and during introducing or transferring the mother solution into the mother solution container. The above atmospheric pressure may be at least 300 mbar, or 350 mbar or 400 mbar and up to 450 mbar or 500 mbar.

The purging of the mother solution container with an inert gas before and during introducing or transferring of the mother solution into the mother solution container reduces radiolytic degradation of the components comprised in the mother solution container.

The transfer from the single container of step (2) into the mother solution container of step (3) may be effected by way of the pressure above atmospheric pressure or by way of syringes.

In step (3) recovering the radionuclide complex may comprise using water-for-injection (WFI) for rinsing, i.e. WFI is added to the single container of step (2) after completion of the reaction and the solution formed in the single container is introduced or transferred into the mother solution container as described above. This ensures complete (or almost complete) transfer of the solution comprising the radionuclide complex, while maintaining a relatively high volumetric activity concentration.

In step (3) the mother solution comprises a nitrogen concentration of up to 20 ml/L at 25 degrees Celsius or an argon concentration of up to 60ml/L at 25 degrees Celsius due to the purging with nitrogen and argon, respectively. The mother solution may comprise a nitrogen concentration in a range of 3 to 20 ml/L 25 degrees Celsius, preferably 5 to 15, more preferably 10 to 15 ml/L at 25 degrees Celsius. The mother solution may comprise an argon concentration of 3 to 60 mL/L at 25 degrees Celsius, preferably 10 to 50, more preferably 20 to 40 mL/L at 25 degrees Celsius.

In step (3) the mother solution comprises a nitrogen concentration of up to 20 mg/L at 25 degrees Celsius or an argon concentration of up to 60 mg/L at 25 degrees Celsius due to the purging with nitrogen and argon, respectively. The mother solution may comprise a nitrogen concentration in a range of 3 to 20 mg/L 25 degrees Celsius, preferably 5 to 15, more preferably 10 to 15 mg/L at 25 degrees Celsius. The mother solution may comprise an argon concentration of 3 to 60 mg/L at 25 degrees Celsius, preferably 10 to 50, more preferably 20 to 40 mg/L at 25 degrees Celsius.

In step (2) reacting the target binding organic molecule with the ¹⁷⁷Lu(III) ions at below atmospheric pressure to obtain the radionuclide complex may be carried out over 2 to 15 minutes, preferably 4 to 10 minutes, more preferably 5 min ± 0.5 min.

In step (2) reacting the target binding organic molecule with the ¹⁷⁷Lu(III) ions at below atmospheric pressure to obtain the radionuclide complex may be carried out at 80 to 100 degrees Celsius, preferably 90 to 98 degrees Celsius, more preferably 94 °C ± 4 °C. Generally, temperatures lower than 90 degrees Celsius do not ensure quantitative labelling yields.

A mixture volume at the step (2) of reacting the target binding organic molecule with the 177 Lu(III) ions at below atmospheric pressure to obtain the radionuclide complex may be 15 to 19 ml.

A final volume containing the radionuclide complex after the step (3) of recovering may be between 20 to 23 ml.

The process of the present disclosure comprising steps (1), (2) and (3) presents the technical advantage that a mother solution is obtained which comprises a radionuclide complex at a volumetric activity which has up to now not been achieved by methods of the prior art, while keeping radiolytic degradation at a minimum. The process facilitates the capturing of a higher total radioactivity originating from the radionuclide complex in the same volume of mother solution compared to published methods of e.g. the applicant. Consequently, after formation of the dispensing solution in step (5) a significantly higher number of individual patient doses (ready-for-use) is provided by the process as compared to the prior art. Therefore, the process as disclosed herein affords a higher number of patient doses per given time unit than methods of the prior art. It therefore contributes to meeting the globally growing need for radiochemicals used in radiotherapy and radiodiagnosis.

The process described herein with DOTATOC or DOTATATE as the target binding organic molecule facilitates production of radionuclide complexes having a total activity higher than 185 GBq (5 Ci) in a volume of mother solution of 20 to 23 ml. For example, an embodiment of the process carried out at a total activity of 296 GBq (8 Ci) in the same volume would lead to ca. 59 to 74 patient doses of ¹⁷⁷Lu-DOTATOC or ¹⁷⁷Lu-DOTATATE (ready-for-use), considering that a single patient dose (ready for use) of ¹⁷⁷Lu-DOTATOC or ¹⁷⁷Lu-DOTATATE would typically comprise a total activity between 4 and 5 GBq (e.g. about 4.7 GBq). In contrast, a process of the prior art that only allows for the processing of a total activity of 148 GBq (4 Ci) in the same volume would only afford ca. 29 to 37 patient doses (ready-for-use).

As a specific example, a therapeutic dose of ¹⁷⁷Lu-DOTA-TATE for the treatment of somatostatin receptor positive gastroenteropancreatic neuroendocrine tumors comprises a total radioactivity of 7,400MBq at the date and time of infusion, typically within a final adjusted volume between 20.5mL and 25.0mL. The process as disclosed herein when processing a total activity of 296 GBq (8 Ci) in the mother solution, would lead to 40 patient doses (ready-for-use), whereas a process of the prior art that allows the processing of only a total activity of 148 GBq (4 Ci) in the same volume would afford only 20 patient doses (ready-for-use).

As stated above, the processes disclosed herein keep radiolytic degradation at a minimum, so that the requirements of radiochemical purity (RCP) are fulfilled. Also, the process provide for a high yield of radiolabelling.

In an embodiment the process may further comprise the steps:
(4) diluting the mother solution of step (3) with a dilution solution to obtain a dispensing solution at a defined volumetric activity,
(5) dispensing said dispensing solution into individual patient dose units.

Said dilution solution may comprise:
(3) a stabilizer against radiolytic degradation, preferably ascorbic acid or salts thereof,
(4) a sequestering agent, preferably DTPA, and
(5) optionally an isotonic agent, preferably NaCl.

The features of the stabilizer, sequestering agent and isotonic agent are as described above in the section mother solution container.

The steps (4) and (5) afford individual patient doses which are destined for subsequent administration to a patient without further material change. The individual patient doses comprise a volumetric activity required for therapeutic or diagnostic purposes.

The defined volumetric activity of the dispensing solution may be adjusted to provide individual patient dose units having a volumetric activity of 1000 MBq/mL ± 5% for 177Lu-PSMA-617 and 370 MBq/mL ± 5% for 177Lu-DOTA-TATE.

The process of the present disclosure may be advantageously used for the synthesis of ¹⁷⁷Lu-DOTA-TATE (¹⁷⁷Lu-oxodotreotide), especially for production of a mother solution which is used for the production of ¹⁷⁷Lu-DOTA-TATE individual patient doses (ready-to-use).

In a specific embodiment of the process the dispensing solution obtained in above step (4) comprises
(A) 177Lu-DOTA-TATE in an activity in the range from 296 to 444 MBq/mL, preferably 333 to 407 MBq/ml, more preferably about 351 to 389 MBq/ml, most preferably about 370 MBq/mL (10 mCi/mL);
(B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
(C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
(D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
(E) ascorbic acid or a salt thereof in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
(F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
(G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
(H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.

The process of the present disclosure may be advantageously used for the synthesis of ¹⁷⁷Lu-PSMA-617, especially for production of a mother solution which is used for the production of ¹⁷⁷Lu-PSMA-617 individual patient doses (ready-to-use).

In another specific embodiment of the process the dispensing solution obtained in above step (4) comprises
(A) **17⁷Lu-PSMA-617** in an activity in the range from 800 to 1200 MBq/ml, preferably 900 to 1100 MBq/ml, more preferably 950 to 1050 MBq/ml, most preferably about 1000 MBq/mL (27 mCi/mL);
(B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
(C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
(D) gentisic acid or a salt thereof in a concentration in the range from 0.3 to 1.0 mg/mL, preferably 0.3 to 0.5 mg/mL, more preferably 0.31 to 0.47 mg/mL, even more preferably 0.35 to 0.43 mg/mL, even more preferably 0.37 to 0.41, most preferably about 0.39 mg/mL with regard to the free acid;
(E) ascorbic acid or a salt thereof in a concentration in the range from 20 to 52.5 mg/mL, preferably 47.5 to 52.5 mg/mL, even more preferable 48 to 52 mg/mL, most preferably about 50 mg/mL with regard to the sodium salt;
(F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

### Implementation of the process steps

The process described above may be implemented in a sealed device comprising a central piece of pipe to which an inlet for inert gas, and an adaptor for applying reduced pressure to the pipe are connectable. Also, containers comprising the reactants described above and water-for-injection are provided and connected to the central piece of pipe. Moreover, containers suitable to receive the reaction solution and the mother solution are provided and connected to the central piece of pipe. All connections to the central piece of pipe are equipped with valves, so that directed transfer of solutions to and from containers is possible, effected by the application of reduced pressure or pressure of an inert gas, depending on the individual step. Transfers may alternatively be effected by the use of syringes connectable to containers and the central piece of pipe via adaptors. All pieces of equipment are made of materials compatible with the reagents used in the process.

The process described above may be advantageously automated and implemented in a synthesis module employing a single use kit cassette.

For example, a single use kit cassette is installed on the front of a synthesis module which contains a fluid pathway (tubing), a reactor vial and sealed reagent vials. The disposable cassette components are made of materials specifically chosen to be compatible with the reagents used in the process. In particular, the components are designed to minimize potential leaching from surfaces in contact with the fluids of the process while maintaining mechanical performance and integrity of the cassette.

Preferably, the process is fully automated and takes place within a computer assisted system.

A typical kit cassette may include
(1) a reaction vial (reactor),
(2) connections for incoming and outgoing fluids,
(3) spikes for connecting reagent vials, and,
(4) optionally, solid phase cartridges.

The skilled person may adapt commercially available kit cassettes used for the preparation of radiopharmaceuticals such as fluorine-18 labeled radiopharmaceuticals.

In specific embodiments, the synthesis module (and kit cassette?) comprise the following:
(i) at a first position, a needle is placed for inserting in the top of a first vial containing the radioactive ¹⁷⁷Lu(III) solution,
(ii) at a second position, a needle is placed for inserting in the top of a vial containing a solution comprising the target binding organic moiety linked to a chelating agent,
(iii) at a third position, a bag with water-for-injection is installed, for rinsing steps,
(iv) at a fourth position, the solution comprising one or more stabilizers against radiolytic degradation is installed, and,
(v) at further positions, needles may be placed for inserting in the top of additional vials (e.g. mother solution container) or tubing may be installed for transfer from the synthesis module to a dispensing isolator.

Specific examples of the synthesis module and the kit cassette are described in the Examples.

### Product obtained by the process of manufacturing

The present disclosure also relates to products obtained by the process of manufacturing as described above.

In a specific embodiment, a product is obtained wherein the dispensing solution comprises:
(A) 177Lu-DOTA-TATE in an activity in the range from 296 to 444 MBq/mL, preferably 333 to 407 MBq/ml, more preferably about 351 to 389 MBq/ml, most preferably about 370 MBq/mL (10 mCi/mL);
(B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
(C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
(D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
(E) ascorbic acid or a salt thereof, preferably the sodium salt thereof, in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
(F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
(G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
(H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.

In another specific embodiment, a product is obtained wherein the dispensing solution comprises:
(A) ¹⁷⁷Lu-PSMA-617 in an activity in the range from 800 to 1200 MBq/ml, preferably 900 to 1100 MBq/ml, more preferably 950 to 1050 MBq/ml, most preferably about 1000 MBq/mL (27 mCi/mL);
(B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
(C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
(D) gentisic acid or a salt thereof in a concentration in the range from 0.3 to 1.0, preferably 0.3 to 0.5 mg/mL, more preferably 0.31 to 0.47 mg/mL, even more preferably 0.35 to 0.43 mg/mL, even more preferably 0.37 to 0.41, most preferably about 0.39 mg/mL with regard to the free acid;
(E) ascorbic acid or a salt thereof, preferably the sodium salt thereof, in a concentration in the range from 20 to 52.5 mg/mL, preferably, 47.5 to 52.5 mg/mL, more preferably 48 to 52 mg/mL, most preferably about 50 mg/mL with regard to the sodium salt;
(F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

### Aqueous pharmaceutical solutions

The present disclosure also relates to aqueous pharmaceutical solutions. In a specific embodiment the present disclosure provides the following aqueous solution:
(A) ¹⁷⁷Lu-PSMA-617 in an activity in the range from 800 to 1200 MBq/ml, preferably 900 to 1100 MBq/ml, more preferably 950 to 1050 MBq/ml, most preferably about 1000 MBq/mL (27 mCi/mL);
(B+C) a buffer to provide a pH value of the solution in the range of 4.5 to 7.0;
(D) gentisic acid or a salt thereof in a concentration in the range from 0.3 to 1.0, preferably 0.3 to 0.5, more preferably 0.31 to 0.47 mg/mL, even more preferably 0.35 to 0.43 mg/mL, even more preferably 0.37 to 0.41, most preferably about 0.39 mg/mL with regard to the free acid;
(E) ascorbic acid or a salt thereof, preferably the sodium salt thereof, in a concentration in the range from 20 to 52.5 mg/mL, preferably 47.5 to 52.5 mg/mL, even more preferably 48 to 52 mg/mL, most preferably about 50 mg/mL with regard to the sodium salt;
(F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

In another specific embodiment the present disclosure provides the following aqueous solution:
(A) ¹⁷⁷Lu-PSMA-617 in an activity in the range from 800 to 1200 MBq/ml, preferably 900 to 1100 MBq/ml, more preferably 950 to 1050 MBq/ml, most preferably about 1000 MBq/mL (27 mCi/mL);
(B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
(C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
(D) gentisic acid or a salt thereof in a concentration in the range from 0.3 to 1.0, preferably 0.3 to 0.5, more preferably 0.31 to 0.47 mg/mL, even more preferably 0.35 to 0.43 mg/mL, even more preferably 0.37 to 0.41, most preferably about 0.39 mg/mL with regard to the free acid;
(E) ascorbic acid or a salt thereof, preferably the sodium salt thereof, in a concentration in the range from 20 to 52.5 mg/mL, preferably 47.5 to 52.5 mg/mL, more preferably 48 to 52 mg/mL, most preferably about 50 mg/mL with regard to the sodium salt;
(F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

In certain embodiments the aqueous solution comprises, contains, or consists of about ¹⁷⁷Lu-PSMA-617 (about 1,000 MBq/mL, about 27 mCi/mL), acetic acid (about 0.30 mg/mL), sodium acetate (about 0.41 mg/mL), gentisic acid (about 0.39 mg/mL), sodium ascorbate (about 50.0 mg/mL), pentetic acid (about 0.10 mg/mL), and water for injection (e.g. q.s. to 1 mL) with the pH range of the solution being from about 4.5 to about 7.0. The term "about" here means ±10% for all ingredients, preferably ±10% for the radioactive ingredient and ±5% for the non-radioactive ingredients.

In further specific embodiments the present disclosure provides any one of the aqueous solutions of the embodiments above, wherein the radiochemical purity (RCP, determined by HPLC) is maintained at ≥ 95% for at least 120 hours when stored at 30°C or below. Accordingly, the shelf-life of the aqueous solutions of the present disclosure is about 120 hours or about 5 days, preferably from the date and time of calibration, with storage conditions of below 30°C (86°F), do not freeze.

In further specific embodiments the present disclosure provides any one of the aqueous solutions of the embodiments above, wherein said solution comprise not more than 5% (w/w) ethanol, preferably not more than 1% ethanol, more preferably does substantially not comprise any ethanol.

In further specific embodiments the present disclosure provides any one of the aqueous solutions of the embodiments above, wherein said solution comprise a total peptide content of from 10 to 20 microgram/mL, preferably 13 to 17 microgram/mL, more preferably 14 to 16 microgram/mL, most preferably 15 microgram/mL.

In certain embodiments, the aqueous solution of the present invention is provided as sterile, preservative-free, clear, colorless to slightly yellow solution. In certain embodiments, the aqueous solution is provided as ready-to-use solution.

The present disclosure further provides individual patient dose unit with about 7.5 to about 12.5 mL content of any one of the aqueous solutions as described in any one of the embodiments above.

Said patient dose unit may be in the form of a vial, e.g. a single-dose vial, e.g. a colorless borosilicate (type I) glass vial, e.g. of about 30 mL size, e.g. closed with a bromobutyl rubber stopper (stopper with silicate filler and inorganic coloring system) and a seal, preferably an aluminium seal, or in the form of a pre-filled syringe or cartridge, e.g. a cartridge that can be loaded into a device for infusion/injection, e.g. a cartridge for a syringe or an infusion system. The dose unit may be provided in a lead shielded container, preferably placed in a plastic sealed container. The dose unit may be shipped in a Type A packaging system (according to the corresponding regulations of the International Air Transport Association (IATA) and International Carriage of Dangerous Good by Road (ADR)). The Type A packaging is designed to meet the radiological protection requirements.

The aqueous solutions of the present disclosure may be first dispensed in a vial and then transferred into a syringe.

The aqueous solutions of the present disclosure may be injected intravenously (IV, by bolus injection or infusion) or intraarterially, or intratumorally. The aqueous solutions of the present disclosure may be administered to the patient by slow intravenous push within approximately 1 to 10 minutes (either with a syringe pump or infusion pump or manually), e.g. via an intravenous catheter that is pre-filled with e.g. 0.9% sterile sodium chloride solution.

The aqueous solutions of the present disclosure may be administered at a dosage/dose of about 7.4 (±10%) GBq (200 (±10%) mCi) every about 6 weeks for up to about 6 doses. E.g. in the context of management of adverse reactions, the dose may be temporarily interrupted (e.g. extending the dosing interval from every about 6 weeks up to every about 7, 8, 9, or 10 weeks), or the dose may be reduced, e.g. by about 20% to about 5.9 (±10%) GBq (160 (±10%) mCi).

When referring to a radioactivity-related value, e.g. 7.4 GBq (200 mCi) of radioactivity, preferably the radioactivity at the date and time of administration is meant.

The Lutetium-177 for the embodiments of the present disclosure may be prepared using two different sources of stable isotopes (either lutetium-176 or ytterbium-176). Lutetium-177 prepared using the stable isotope lutetium 176 is also referred to as "carrier added" (c.a., CA) may contain small amounts of long-lived metastable lutetium-177 (^{177m}Lu) with a half life of 160.4 days. Lutetium-177 prepared using ytterbium-176 is also referred to as "non carrier added" (n.c.a., NCA). In the embodiments of the present disclosure both versions of Lutetium-177, CA as well as NCA, may be used, preferably without changing the other components qualitatively or quantitatively. Preferably, n.c.a. ¹⁷⁷Lu is used.

### EXAMPLES

### Example 1: Production of a sterile, aqueous concentrated solution of ¹⁷⁷Lu-DOTA-TATE

### 1.1 Introduction

The radioactive Drug Substance ¹⁷⁷Lu-DOTA-TATE, also referred hereafter as ¹⁷⁷Lu-DOTAO-Tyr³-Octreotate is produced as a sterile, aqueous concentrated solution (so-called Mother Solution).

Drug Substance synthesis steps are performed in a self-contained closed-system synthesis module which is automated and remotely controlled by GMP compliant software and automated monitoring and recording of the process parameters.

During each production run of the synthesis module, a single use disposable kit cassette, containing a fluid pathway (tubing), reactor vial and sealed reagent vials is used. The synthesis module is protected from manual interventions during the production run. The synthesis module is placed in a lead-shielded hot cell providing supply of filtered air.

The synthesis of the Drug Substance (¹⁷⁷Lu-DOTAO-Tyr³-Octreotate) and its formulation into the Drug Product (¹⁷⁷Lu-DOTAO-Tyr³-Octreotate 370 MBq/mL solution for infusion), is part of an automated continuous process which does not allow for isolation and testing of Drug Substance due to its radioactive decay.

The synthesis of the Drug Substance is carried out using MiniAio (Trasis) kit cassette. For the kit assembly (under Grade C), follow Figure 1. The following flow chart shows the chemistry process for the manufacturing of the Drug Substance in the Grade C Hot Cells at 4 Ci and 8 Ci batch size.

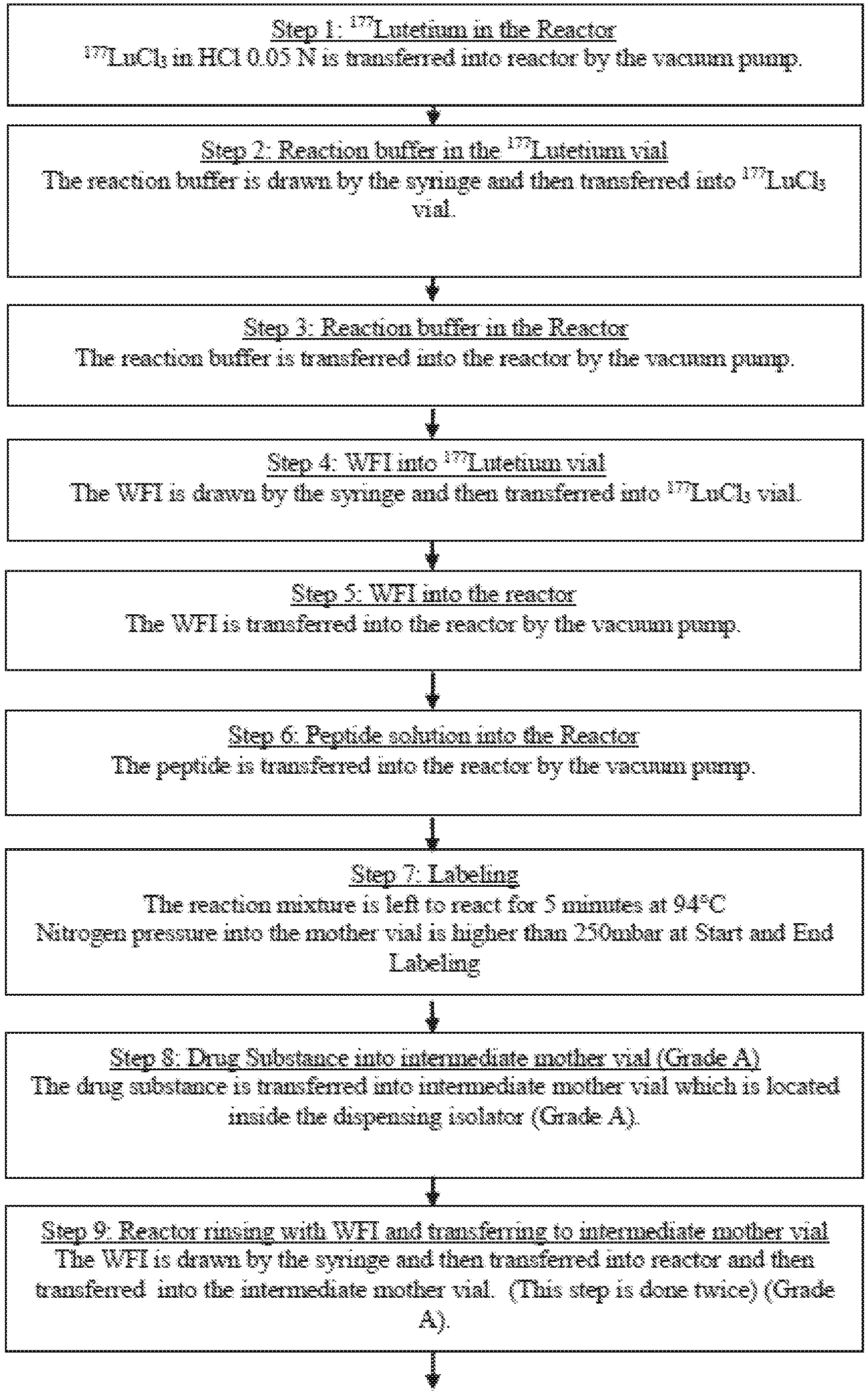

The labeling (step 7) consists of the chelating of ₁₇₇Lu into the DOTA moiety of the DOTA-Tyr₃-Octreotate peptide. The labeling is carried out at 94°C±4°C.

In the reaction mixture DOTA₀-Tyr₃-Octreotate is present in a molar excess respect to the ₁₇₇Lu to ensure acceptable radiochemical labeling yields. The chemical reaction for producing the Drug Substance ₁₇₇Lu-DOTA₀-Tyr₃-Octreotate is illustrated in the following.

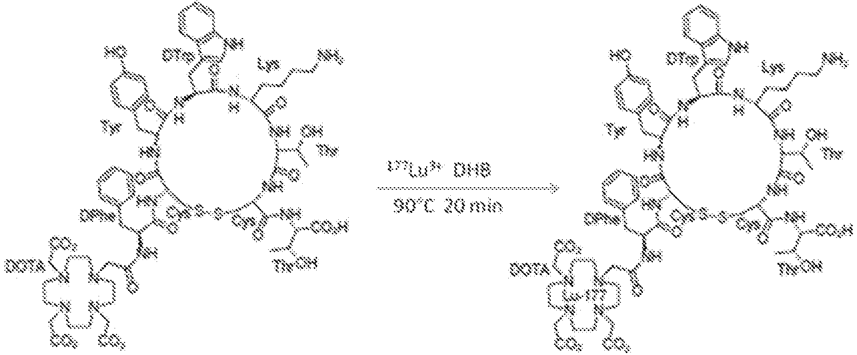

The formulation, sterilizing filtration and dispensing processes of the Drug Product are carried out in the Grade A Dispensing Isolator. The following flow chart shows in the detail the steps for the manufacturing of the Drug Product.

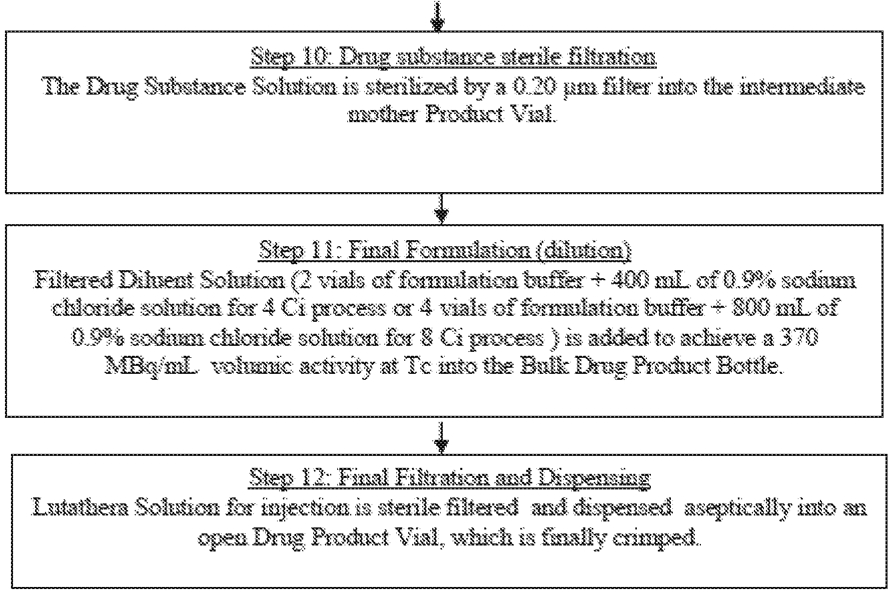

### Preparation of starting materials

The chemical precursors, radioactive precursor and intermediate of drug substance used in the manufacturing process are prepared according to the following Table 1.

**Table 1**

| **Component** | **Method of Preparation** |
|---|---|
| Chemical Precursor of Drug Substance | Solid phase synthesis purification and isolation of **DOTA-TATE (TFA salt) lyophilized,** also called **DOTA-Tyr³-Octreotate)** |
| Radioactive precursor of Drug Substance | Neutron bombardment of enriched Lu-176 in a nuclear reactor to manufacture a **Lu-177 chloride solution** in dilute hydrochloric acid |
| Intermediate of Drug Substance | **Reaction Buffer Lyophilisate (RBL)** containing gentisic acid, and sodium acetate. |

The details of the reaction buffer lyophilisate are provided below in Table 2:

**Table 2**

| **Components** | **Quantity (mg/vial)** | **Quan** | **tity/batch Function** |
|---|---|---|---|
| Gentisic acid | 157.5 mg | 39.38 g | Radiation Stability Enhancer |
| Acetic acid | 120.2 mg | 28.76 mL | pH adjuster |
| Sodium acetate | 164.0 mg | 41.00 g | pH adjuster |
| Water for injections | q.s up to **4 mL** | up to **1000 mL** | Solvent |

### Preparation of the synthesis module and kit cassette

The manufacturing process has been validated using two different Lu-177 chloride batch sizes, 74.0 GBq ± 20 % (2 Ci ± 20 %) or 148.0 GBq ± 20 % (4 Ci ± 20 %).

The synthesis is carried out using a single use disposable kit cassette installed on the front of the synthesis module which contains the fluid pathway (tubing), reactor vial and sealed reagent vials.

### Kit cassette for MiniAIO synthesis module

The kit cassette is ready-to-use.

### Step 1c: Reaction Buffer Lyophilisate dissolution

Before its use in the Drug Substance synthesis, Reaction Buffer Lyophilisate (RBL) is reconstituted by Drug Substance manufacturing site by dissolution with water for injection (WFI) to obtain Reaction Buffer solution.

Reconstitution is carried out immediately before the start of the synthesis.

To dissolve the RBL:
For 74 GBq batch size (2 Ci batch size): one vial of RBL is reconstituted with 2 mL of WFI using a sterile, disposable syringe.

For 148 GBq batch size (4 Ci batch size): two vials of RBL are reconstituted with 2 mL of WFI per vial using a sterile, disposable syringe. The content of one solubilised Reaction Buffer vial is transferred into the other one using a sterile disposable syringe, and mixed up in order to obtain one vial containing 4 mL of product.

After reconstitution, the composition of Reaction Buffer is as described in Table 4.

**Table 4: Reaction Buffer compositions after reconstitution**

| **Components** | **Acceptance Limit** | **Reference to standards** | **Function** |
|---|---|---|---|
| Gentisic Acid | 157.5 ± 5 % mg | In-house | Radiation Stability Enhancer |
| Acetic Acid | 120.2 ± 5% mg | In-house | pH adjuster |
| Sodium Acetate | 164.0 ± 5 % mg | Ph.Eur. 0411/USP | pH adjuster |
| Water for Injection (WFI) | qs 2.00 mL | Ph.Eur. 0169/ USP | Solvent |

### 1.2 Step 1d : DOTA-Tyr³-Octreotate Dissolution (chemical precursor)

DOTA-Tyr³-Octreotate is provided as a dry powder in vial. Each vial is of 2 mg of DOTA-Tyr³-Octreotate. Before the synthesis reaction, DOTA-Tyr³-Octreotate is dissolved in water for injection (WFI).

To dissolve the DOTA-Tyr³-Octreotate:
- For 74 GBq batch size (2 Ci batch size): one vial of DOTA-Tyr³-Octreotate is reconstituted with 2 mL of WFI using a sterile, disposable syringe.
- For 148 GBq batch size (4 Ci batch size): two vials of DOTA-Tyr³-Octreotate are reconstituted with 2 mL of WFI per vial. The content of one solubilised DOTA-Tyr³-Octreotate vial is transferred into the other one using a sterile disposable syringe, and mixed up in order to obtain one vial containing 4 mL of product.

### Step 5: Installation of starting material on the kit cassette

Reaction Buffer solution, WFI and precursors are installed on the corresponding cassette positions according to the synthesis module used. The installations are performed in a Grade C environment.

### Step 6: Transfer of Lu-177 chloride solution, Reaction Buffer solution and DOTA-Tyr³-Octreotate solution into the reactor

The synthesis is initiated by pushing the "start synthesis" button on the synthesis module PC control software program. The first step of the synthesis consists of the automated transfer of all components needed for the labeling into the cassette reactor.

Radioactive and chemical Drug Substance precursors and Reaction Buffer solution are transferred into the reactor in the following order:
1. Lu-177 chloride solution
2. Reaction Buffer solution
3. DOTA-Tyr³-Octreotate solution

The Lu-177 chloride solution is drawn into the reactor when the valves (positions 5 and 6 of the GE cassette or positions 1 and 2 of the MiniAIO cassette), are opened and negative pressure is applied to the reactor.

The Lu-177 chloride solution is highly concentrated and therefore incomplete transfer of the solution into the reactor 1 can impact the labeling yield. For this reason, the Reaction Buffer solution is added to the Lu-177 chloride solution vial before its transfer into the reactor in order to ensure complete transfer of the Lu-177 chloride solution. Reaction Buffer is transferred into Lu-177 chloride vial using syringe (right 30 mL syringe¹ for TRACERlab MX synthesis module and 30 mL syringe² for MiniAIO synthesis module). From this vial, the solution (Reaction Buffer + Lu-177 residual) is transferred into the reactor by applying negative pressure.

The last step to initiate synthesis of the Drug Substance is the transfer of the DOTA-Tyr³-Octreotate solution to the reactor. This is automatically performed by negative pressure applied to the reactor.

### 1.10 Step 7: Labelling step

The synthetic route is summarized as follows:

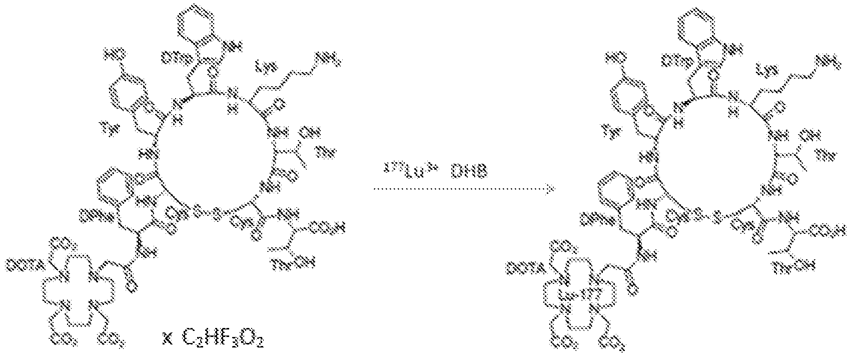

With DHB = gentisic acid (2,5-dihydroxybenzoic acid)

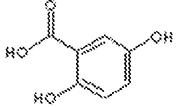

The labeling consists of the chelating of Lu-177 into the DOTA moiety of the DOTA-Tyr³-Octreotate peptide. The labelling is carried out at 94°C (± 4°C) for:
- 5 minutes (± 0.5 minutes) using MiniAIO (TRASIS) synthesis module

In the reactor, DOTA-Tyr³-Octreotate is present in a molar excess respect to Lu-177 to ensure acceptable radiochemical labeling yields (see also Example 2 related to the process optimization).

### 1.11 Step 8: Transfer and first filtration of Drug Substance (prefiltration)

Once the synthesis is finished in the synthesis module, ¹⁷⁷Lu-DOTA⁰-Tyr³-Octreotate Mother Solution obtained is sterilized a first time using a sterilizing filter connected to the extension sterile cable. During the filtration, the ¹⁷⁷Lu-DOTA⁰-Tyr³-Octreotate Mother Solution is automatically transferred by positive nitrogen pressure from the synthesis hot-cell (Grade C) into the dispensing isolator Grade A by the extension sterile cable and is collected in an intermediate 30 mL sterile vial. A vent filter with a microlance needle is used to equilibrate pressure in the intermediate 30 mL sterile vial.

The cassette and the reactor are rinsed 3 times with 3 mL of water for injection each time, in order to recover ¹⁷⁷Lu-DOTA⁰-Tyr³-Octreotate remaining in the lines.

The volume of ¹⁷⁷Lu-DOTA⁰-Tyr³-Octreotate Mother Solution at the end of the transferring process is:
- For the 74 GBq batch size (2 Ci batch size): ≥ 13.0 mL
- For the 148 GBq batch size (4 Ci batch size): ≥ 19.0 mL
- For the 8 Ci batch size: : ≥ 19.0 mL

The volume and the radioactivity of the ¹⁷⁷Lu-DOTA⁰-Tyr³-Octreotate Mother Solution are controlled at the end of the synthesis and monitored. The synthesis yield is calculated.

### 1.13 Results

| Batch | Date | Batch Size 8Ci | Mother Solution, volumetric activity (GB/mL) | Nitrogen Flush of Mother Vial | Holding Time Mother Vial | RCP Texp 5mL (≥ 95.00%) | RCP Texp 20mL (≥ 95.00%) |
|---|---|---|---|---|---|---|---|
| 1 | 24/10/18 | 8 Ci | 15,6 | NO | 1h | 91,84 | 95,75 |
| 2 | 14/11/18 | 8 Ci | 16,0 | NO | 1h | 92,10 | 95,48 |
| 3 | 28/11/18 | 8 Ci | 14,3 | NO | 1h | 82,39 | 93,59 |
| 8 | 22/01/19 | 8 Ci | 6,3 | YES | 1h | 96,78 | 97,90 |
| 9 | 12/02/19 | 8 Ci | 14,6 | YES | 1h | 96,71 | 96,83 |
| 10 | 19/02/19 | 8 Ci | 14,2 | YES | 0 min | 96,44 | 97,39 |

| | | | | | | | |
|---|---|---|---|---|---|---|---|
| Mother Solution, volumetric activity: measured after rinsing Texp: Expiration Time of Product shelf life ("5ml" indicates ca. 5 ml solution in ca. 30 ml vial; "20ml" indicates ca. 20 ml in 30 ml vial) RCP: Radiochemical purity (determined by HPLC) | | | | | | | |

The above table shows that during Lutathera 8Ci manufacturing process development, the experimental tests showed very good results when nitrogen was used to completely degas the mother solution vial to reduce the radiolysis of the product over shelf-life. Specifically, during the mother solution vial degassing step the air in the vial, containing oxygen that is assumed to be at least partly responsible for radiolysis, is replaced with a nitrogen flush to the extent possible.

The difference in values between Texp 5ml and Texp 20 ml data originates from the difference in size of the headspace volume with the Texp 5 ml experiment having a much larger headspace volume.

The results above clearly indicate that completely degassing a mother solution vial and optionally holding a vial under inert gas atmosphere leads to superior radiochemical purity when measured at the end of the product shelf life.

### Example 2: 177Lu-PSMA-617 manufacturing

The synthesis of the Drug Substance at 200 GBq or 400 GBq scale is carried out using MiniAio (Trasis) kit cassette. For the kit assembly (under Grade C), follow Figure 2. The following flow chart shows the chemistry process for the manufacturing of the Drug Substance in the Grade C Hot Cells.

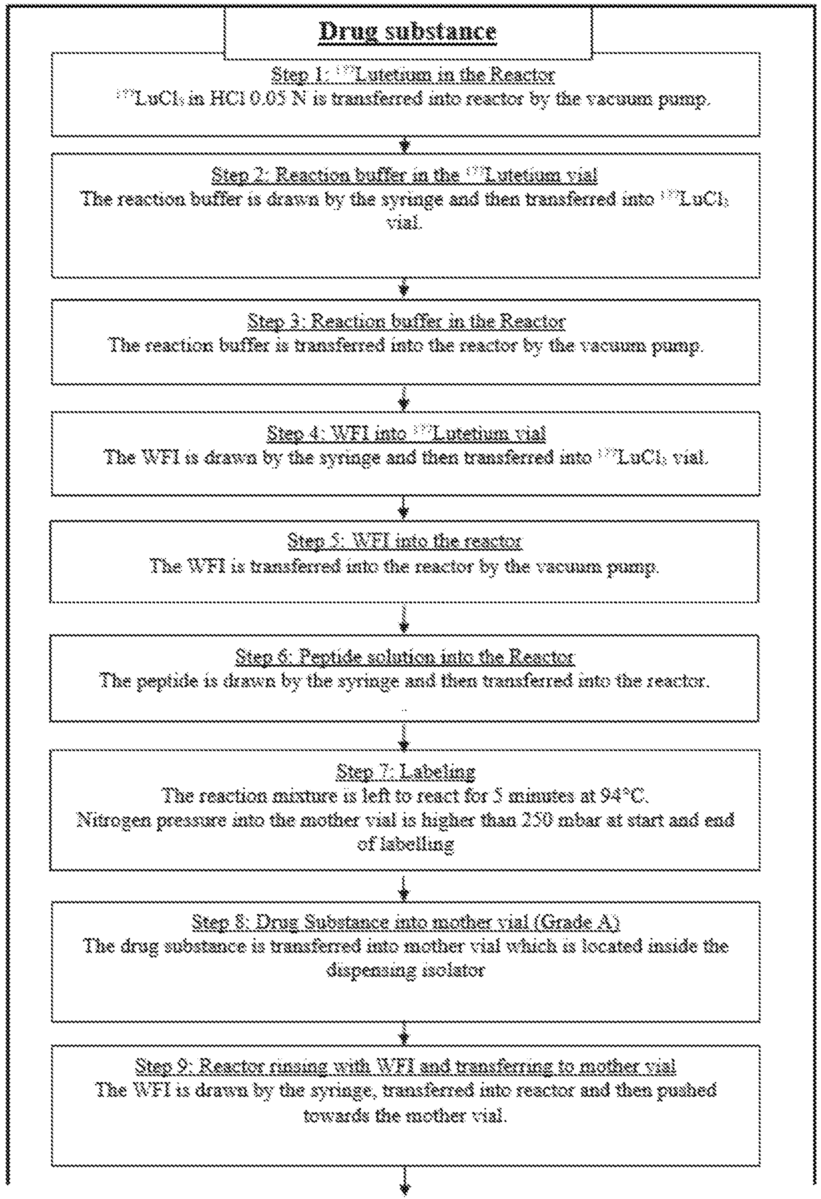

The labeling (step 7) proceeds by chelation of ₁₇₇Lu into the DOTA moiety of the PSMA-617. The labeling is carried out at 94°C ± 4°C for 5 ± 0.5 minutes.

In the reaction mixture DOTA-PSMA is present in a molar excess respect to the 177Lu to ensure acceptable radiochemical labeling yields. The chemical reaction for producing the Drug Substance 177Lu-DOTA-PSMA is illustrated in the figure below:

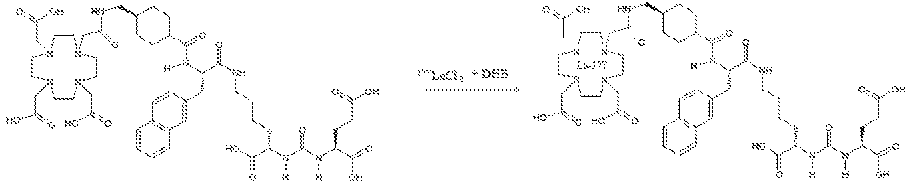

Drug product formulation, sterilizing filtration and dispensing are carried out in the Grade A Dispensing Isolator. The following flow chart shows in detail the steps for the manufacturing of the Drug Product.

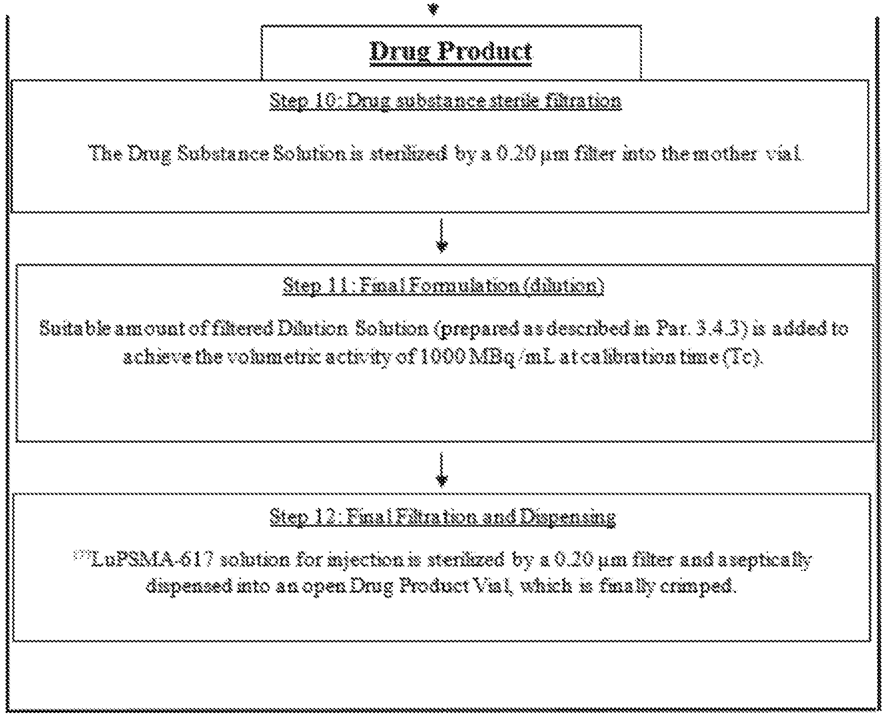

The dilution solution is prepared by dissolving the appropriate amounts of sodium ascorbate and pentetic acid (DTPA) in water for injection (WFI).

The following table provides additional experimental information to examples 1 and 2.

| | **DOTATATE** | | | **PSMA-617** | |
|---|---|---|---|---|---|
| **Batch size** (validated with ±20%) | **2 Ci, 74GBq** | **4 Ci, 148 GBq** | **8 Ci, 296 GBq** | **200 GBq, 5.4 Ci** | **400 GBq, 10.8 Ci** |
| ¹⁷⁷LuCl₃ solution (in 0.05 N HCl) | 74 GBq in 1.5 mL | 148 GBq in 2.5 mL | 296 GBq in 4.5 mL | 200 GBq in 2 mL | 400 GBq in 4 mL |
| Reaction buffer solution* | 2.0 mL | 4.0 mL | 8.0 mL | 4.0 mL but only used: 2.0mL | 4.0 mL |
| Dotatate/PSMA-617 solution (in WFI) | 2 mg in 2.0 mL | 4 mg in 4.0 mL | 8 mg in 4.0 mL | 3 mg in 3.0 mL | 6 mg in 6.0 mL |
| Total volume at radiolabeling | 5.5 mL | 10.5 mL | 16.5-17.5 mL | 7-10.5 mL | 14-15.5 mL |
| RADIOLABELING REACTION | | | | | |
| *Rinsed 2-3 x with 2-3 mL WFI = 6-9 mL | ~9 mL | ~9 mL | ~6mL | ~8mL | ~6mL |
| MOTHER SOLUTION | | | | | |
| Specification for mother solution | 44.4 GBq ≥ in ≥ 13.0 mL | ≥ 89.0 GBq in ≥ 19.0 mL | ≥ 177.0 GBq in ≥ 19.0 mL | ≥ 120.0 GBq in ≥ 16.0 mL | ≥ 240.0 GBq in ≥ 16.0 mL |
| Typical value of activity of mother solution | | | 310-320 GBq | | |

| | | | | | |
|---|---|---|---|---|---|
| *Reaction buffer solution for **177Lu-DOTATATE:** Gentisic acid: 157.5 mg Acetic acid: 120.2 mg Sodium acetate: 164.0 mg Water WFI: ad 2 mL *Reaction buffer solution for **177Lu-PSMA-617** Gentisic acid: 157.5 mg Acetic acid: 120.2 mg Sodium acetate: 164.0 mg Water WFI: ad 4 mL | | | | | |

Holding time for handling the mother solution: 60 min.

Typical yield of the process: 92-95%.

The theoretical batch formula of the bulk drug product ¹⁷⁷Lu-PSMA-617 solution is described in the following table. Independently of the size of the batch of drug product, the ratios of acetic acid, sodium acetate, gentisic acid, sodium ascorbate pentetic acid and water for injections are maintained.

Batch formula for 177Lu-PSMA-617 solution for injection/infusion:

| **Ingredients** | **Amount per 200 GBq batch size (5.4 Ci)** | **Amount per 400 GBq batch size (10.8 Ci)** |
|---|---|---|
| ¹⁷⁷Lu-PSMA-617 | 1000 MBq/mL ± 5 % at Tc | |
| Acetic acid | 60.1 mg | 120.2 mg |
| Sodium acetate | 82.0 mg | 164.0 mg |
| Gentisic acid | 78.8 mg | 157.5 mg |
| Sodium ascorbate | 10.0 g | 20.0 g |
| Pentetic acid (DTPA) | 20.0 mg | 40.0 mg |
| Water for injection(s) | Up to 200 mL | Up to 400 mL |

| | | |
|---|---|---|
| Tc: Calibration time; DTPA: Diethylenetriaminepentaacetc acid | | |

A batch size can contain 1- 40 customer vials according to the batch size.

The composition of the drug product 177Lu-PSMA-617 solution for injection/infusion per mL of solution is described in the following table.

| **Ingredient** | **Amount (per mL)³** | **Function** |
|---|---|---|
| ¹⁷⁷Lu-PSMA-617 | 1000 MBq ± 5% (Tc) | Drug substance |
| Acetic acid | 0.30 mg | pH adjuster |
| Sodium acetate | 0.41 mg | pH adjuster |
| Gentisic acid | 0.39 mg | Radiation stability enhancer |
| Sodium ascorbate | 50.0 mg | Radiation stability enhancer |
| Pentetic acid (DTPA) | 0.10 mg | Sequestering agent |
| Water for injection(s) | q.s to 1 mL² | Solvent |

| | | |
|---|---|---|
| Tc: calibration time = end of production, 2: includes all water, also the small amounts of water that may be left over from the sterilization process. 3: calculated and rounded values. | | |

As consequence of the natural decay of the radionuclide the total radioactivity and the radioconcentration (volumetric activity) of the drug product change over time. The composition of the drug product per single dose taking as reference the minimum (7.5 mL) and the maimum (12.5 mL) filling content is described in the following table.

| **Ingredient** | **Amount per 7.5 mL (minimum volume)** | **Amount per 12.5 mL (maximum volume)** | **Function** |
|---|---|---|---|
| ¹⁷⁷Lu-PSMA-617 | 1000 MBq/mL | ± 5% (Tc) | Drug substance |
| Acetic acid | 2.25 mg | 3.75 mg | pH adjuster |
| Sodium acetate | 3.08 mg | 5.13 mg | pH adjuster |
| Gentisic acid | 2.93 mg | 4.88 mg | Radiation stability enhancer |
| Sodium ascorbate | 375.0 mg | 625.0 mg | Radiation stability enhancer |
| Pentetic acid (DTPA) | 0.75 mg | 1.25 mg | Sequestering agent |
| Water for injection(s) | q.s to 7.5 mL² | q.s to 12.5 mL² | Solvent |

| | | | |
|---|---|---|---|
| Tc: calibration time = end of production, 2: includes all water, also the small amounts of water that may be left over from the sterilization process. | | | |

**The invention is further described by the following numbered clauses:**
1. A reaction solution for radiolabeling a target binding organic molecule with 177Lu(III) ions, wherein said reaction solution comprises:
   (1) 177Lu(III) ions in a volumetric activity of at least 17 GBq/mL,
   (2) a target binding organic molecule comprising a target binding organic moiety linked to a chelating moiety suitable for chelating Lu(III) ions, and
   (3) one or more stabilizers against radiolytic degradation.
2. The reaction solution according to clause 1, comprising an oxygen concentration lower than 3 mg/L at 25 degrees Celsius.
3. A mother solution for preparing a dispensing solution comprising 177Lu radiolabeled target binding organic molecule, wherein said mother solution comprises:
   (1) 177Lu(III) ions in a volumetric activity of at least 10 GBq/mL,
   (2) a radionuclide complex formed by a target binding organic molecule comprising a target binding organic moiety linked to a chelating moiety and the 177Lu(III) ions,
   (3) one or more stabilizers against radiolytic degradation, and
   (4) an oxygen concentration lower than 3 mg/L at 25 deg C.
4. The mother solution according to clause 3, comprising a nitrogen concentration of up to 20 ml/L at 25 degrees Celsius or an argon concentration of up to 60ml/L at 25 degrees Celsius.
5. The reaction solution or mother solution according to any of clauses 1 to 4, wherein the stabilizer against radiolytic degradation comprises gentisic acid or a salt thereof.
6. The reaction solution or mother solution according to clause 5, wherein the concentration of gentisic acid or a salt thereof is from 5 to 15 mg/mL.
7. The reaction solution or mother solution according to clause 6, wherein for the target binding organic moiety being a somatostatin receptor-binding peptide the concentration of gentisic acid or salt thereof is from 10 to 15 mg/mL.
8. The reaction solution or mother solution according to clause 6, wherein for the target binding organic moiety being a PSMA-binding peptide the concentration of gentisic acid or salt thereof is from 5 to 10 mg/ml.
9. The reaction solution or mother solution according to any of clauses 1 to 8, wherein said reaction or mother solution comprises ascorbic acid or a salt thereof in not more than 5% (w/w), preferably not more than 2%, even more preferably not more than 1%, even more preferably does not comprise ascorbic acid (substantially 0%).
10. The reaction solution or mother solution according to any of clauses 1 to 9, wherein said reaction or mother solution comprises ethanol in not more than 5%(w/w), preferably not more than 2%, even more preferably not more than 1%, even more preferably does not comprise ethanol (substantially 0%).
11. The reaction solution or mother solution according to any of clauses 1 to 7, 9 or 10, wherein the target binding organic moiety is a somatostatin receptor binding peptide.
12. The reaction solution or mother solution according to clause 11, wherein the somatostatin receptor binding peptide linked to a chelating moiety comprises DOTA.
13. The reaction solution or mother solution according to any of clauses 1 to 6, 8, 9 or 10, wherein the target binding organic moiety is a PSMA- binding peptide.
14. The reaction solution or mother solution according to clause 13, wherein the PSMA receptor binding peptide linked to a chelating moiety comprises DOTA.
15. The reaction solution or mother solution according to any of clauses 1 to 14, wherein the target binding organic molecule is in molar excess to the 177 Lu (III) ions.
16. The reaction solution or mother solution according to any of clauses 1 to 15, wherein the target binding organic molecule is in molar excess to the group of all Lu (III) ions including 177 Lu (III) ions, 176 Lu(III) ions, 175 Lu(III), and metastable 177m Lu(III) ions present when carrier-added 177 Lu(III) is used as a source of 177 Lu(III).
17. The reaction solution or mother solution according to clause 15 or 16, wherein the molar ratio is at least 1.2, preferably between 1.5 and 3.5.
18. The mother solution according to any of clauses 4 to 17, wherein the nitrogen concentration is 3 to 20 ml/L at 25 degrees Celsius.
19. The mother solution according to any of clauses 4 to 17, wherein the argon concentration is 3 to 60 ml/L at 25 degrees Celsius.
20. The reaction solution or mother solution according to any of clauses 1 to 19, comprising a pharmaceutically acceptable buffer to provide a pH in the range of 2 to 8, which is suitable for the reaction between the 177Lu(III) ions and the target binding organic molecule.
21. The reaction solution or mother solution according to clause 20, wherein the pharmaceutically acceptable buffer provides a pH of 4 to 6.
22. The reaction solution or mother solution according to clause 20 or 21, wherein the pharmaceutically acceptable buffer comprises an acetate buffer, a citrate buffer or a phosphate buffer.
23. The reaction solution or mother solution according to any of clauses 20 to 22, wherein the pharmaceutically acceptable buffer comprises an acetate buffer.
24. The mother solution according to any of clauses 11, 12, 15 to 23, wherein the radionuclide complex is ¹⁷⁷Lu-DOTA-TOC (¹⁷⁷Lu-edotreotide) or ¹⁷⁷Lu-DOTA-TATE (¹⁷⁷Lu-oxodotreotide), preferably ¹⁷⁷Lu-DOTA-TATE (¹⁷⁷Lu-oxodotreotide), having a volumetric activity of 12 GBq/ml to 17 GBq/ml.
25. The mother solution according to any of clauses 13 to 23, wherein the radionuclide complex is 177Lu PSMA-617 having a volumetric activity of 18 to 19 GBq/ml, wherein PSMA-617 has the structure 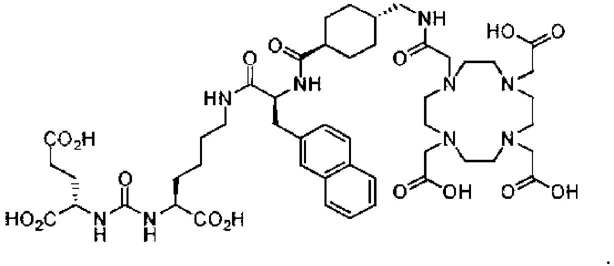
26. A mother solution container for collecting solutions from a radiolabeling reaction, wherein said container comprises:
   (1) a mother solution comprising:
      a. 177Lu(III) ions in a volumetric activity of at least 10 GBq/mL,
      b. a radionuclide complex formed by a a target binding organic molecule comprising a target binding organic moiety linked to a chelating moiety and the 177Lu(III) ions,
      c. one or more stabilizers against radiolytic degradation, and
   (2) a headspace gas volume above the mother solution, wherein said headspace gas volume contains not more than 10 vol% oxygen.
27. The mother solution container according to clause 26, wherein the stabilizer against radiolytic degradation comprises gentisic acid or a salt thereof.
28. The mother solution container according to clause 27, wherein the concentration of gentisic acid or a salt thereof is from 5 to 15 mg/mL.
29. The mother solution container according to clause 28, wherein for the target binding organic moiety being a somatostatin receptor-binding peptide the concentration of gentisic acid or salt thereof is from 10 to 15 mg/mL.
30. The mother solution container according to clause 28, wherein for the target binding organic moiety being a PSMA-binding peptide the concentration of gentisic acid or salt thereof is from 5 to 10 mg/ml.
31. The mother solution container according to any of clauses 26 to 30, wherein the mother solution comprises ascorbic acid or a salt thereof in not more than 5% (w/w), preferably not more than 2%, even more preferably not more than 1%, even more preferably does not comprise ascorbic acid or a salt thereof (substantially 0%).
32. The mother solution container according to any of clauses 26 to 31, wherein the mother solution comprises ethanol in not more than 5%(w/w), preferably not more than 2%, even more preferably not more than 1%, even more preferably does not comprise ethanol (substantially 0%).
33. The mother solution container according to any of clauses 26 to 29, 31, 32 wherein the target binding organic moiety is a somatostatin receptor binding peptide.
34. The mother solution container according to clause 33, wherein the somatostatin receptor binding peptide linked to a chelating moeity comprises DOTA.
35. The mother solution container according to any of clauses 26 to 28, 30 to 32, wherein the target binding organic moiety is a PSMA receptor binding peptide.
36. The mother solution container according to clause 35, wherein the PSMA receptor binding peptide linked to a chelating moeity comprises DOTA.
37. The mother solution container according to any of clauses 26 to 36, wherein the target binding organic molecule is in molar excess to the 177 Lu (III) ions.
38. The mother solution container according to any of clauses 26 to 37, wherein the target binding organic molecule is in molar excess to 177 Lu (III) ions, and in excess to 176 Lu(lll) ions and metastable 177m Lu(lll) ions present when carrier-added 177 Lu(III) is used as a source of 177 Lu(III).
39. The mother solution container according to clause 37 or 38, wherein the molar ratio is at least 1.2, preferably between 1.5 and 3.5.
40. The mother solution container according to any of clauses 26 to 39, wherein said headspace gas volume contains not more than 7 vol% oxygen, preferably not more than 5 vol% oxygen, preferably not more than 3 vol% oxygen, preferably not more than 1 vol% oxygen.
41. The mother solution container according to any of clauses 26 to 40, wherein the mother solution comprises an oxygen concentration lower than 3 mg/L at 25 deg C.
42. The mother solution container according to clause 41, wherein the mother solution comprises a nitrogen concentration of up to 20 ml/L at 25 degrees Celsius or an argon concentration of up to 60ml/L at 25 degrees Celsius.
43. The mother solution container according to clause 42, wherein the nitrogen concentration is 3 to 20 ml/L at 25 degrees Celsius.
44. The mother solution container according to clause 42, wherein the argon concentration is 3 to 60 ml/L at 25 degrees Celsius.
45. The mother solution container according to any of clauses 26 to 44, comprising a pharmaceutically acceptable buffer to provide a pH in the range of 2 to 8, which is suitable for the reaction between the 177Lu(III) ions and the target binding organic moiety linked to a chelating agent.
46. The mother solution container according to clause 45, wherein the pharmaceutically acceptable buffer provides a pH of 4 to 6.
47. The mother solution container according to clause 45 or 46, wherein the pharmaceutically acceptable buffer comprises an acetate buffer, a citrate buffer or a phosphate buffer.
48. The mother solution container according to any of clauses 45 to 47, wherein the pharmaceutically acceptable buffer comprises an acetate buffer.
49. The mother solution container according to any of clauses 33, 34, 37 to 48, wherein the radionuclide complex is ¹⁷⁷Lu-DOTA-TOC (¹⁷⁷Lu-edotreotide) or ¹⁷⁷Lu-DOTA-TATE (¹⁷⁷Lu-oxodotreotide), preferably ¹⁷⁷Lu-DOTA-TATE (¹⁷⁷Lu-oxodotreotide), having a volumetric activity of 12 GBq/ml to 17 GBq/ml.
50. The mother solution container according to any of clauses 35 to 48, wherein the radionuclide complex is 177Lu PSMA-617 having a volumetric activity of 18 to 19 GBq/ml, wherein PSMA-617 has the structure 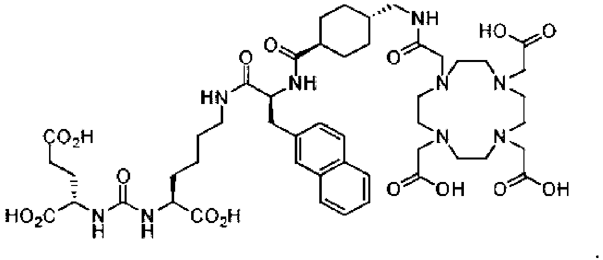
51. The mother solution container according to any of clauses 26 to 50, further comprising:
   (3) a stabilizer against radiolytic degradation, preferably ascorbic acid or salts thereof,
   (4) a sequestering agent, preferably DTPA, and
   (5) optionally an isotonic agent, preferably NaCl.
52. The mother solution container according to any of clauses 26 to 51, comprising
   (A) **177Lu-DOTATATE** in an activity in the range from 296 to 444 MBq/mL, preferably 333 to 407 MBq/ml, more preferably about 351 to 389 MBq/ml, most preferably about 370 MBq/mL (10 mCi/mL);
   (B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
   (C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
   (D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
   (E) ascorbic acid or a salt thereof in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
   (F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
   (G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
   (H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.
53. The mother solution container according to any of clauses 26 to 51, comprising
   (A) 177Lu-PSMA-617 in an activity in the range from 800 to 1200 MGq/ml, preferably 900 to 1100 MBq/ml, more preferably 950 to 1050 MBq/ml, most preferably about 1000 MBq/mL (27 mCi/mL);
   (B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
   (C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
   (D) gentisic acid or a salt thereof in a concentration in the range from 0.31 to 0.47 mg/mL, preferably 0.35 to 0.43 mg/mL, more preferably 0.37 to 0.41, most preferably about 0.39 mg/mL with regard to the free acid;
   (E) ascorbic acid or a salt thereof in a concentration in the range from 40 to 60 mg/mL, preferably 45 to 55 mg/mL, more preferably 47.5 to 52.5 mg/mL, most preferably about 50 mg/mL with regard to the sodium salt;
   (F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.
54. A Process for manufacturing a radiopharmaceutical solution comprising the steps:
   (1) providing the reaction solution according to any of clauses 1, 2, 5 to 17, 20 to 23,
   (2) reacting the a target binding organic molecule comprising a target binding organic moiety linked to a chelating moiety with the 177 Lu(III) ions at below atmospheric pressure to obtain the radionuclide complex in a single container for radiolabeling.
55. The process according to clause 54 for manufacturing a radiopharmaceutical solution, further comprising the step:
   (3) recovering the radionuclide complex to obtain a mother solution.
56. The process for manufacturing according to clause 55, wherein the step (3) of recovering the radionuclide complex comprises recovering under an inert gas atmosphere.
57. The process for manufacturing according to clause 55 or 56, wherein the step (3) of recovering the radionuclide complex comprises introducing or transferring the mother solution into a single container affording a mother solution container.
58. The process for manufacturing according to clause 57, wherein the step (3) of recovering the radionuclide complex comprises purging the mother solution container with an inert gas before and during introducing or transferring the mother solution into the mother solution container.
59. The process for manufacturing according to clause 58, wherein the step (3) of recovering the radionuclide complex comprises purging the mother solution container with an inert gas at a pressure of at least 250 mbar above atmospheric pressure before and during introducing or transferring the mother solution into the mother solution container.
60. The process for manufacturing according to any of clauses 54 to 59, wherein the step (2) of obtaining the radionuclide complex in a single container for radiolabelling comprises an oxygen concentration lower than 3 mg/L at 25 degrees Celsius.
61. The process for manufacturing according to any of clauses 56 to 60, wherein in step (3) the inert gas atmosphere is provided by nitrogen or argon.
62. The process for manufacturing according to clause 61, comprising a nitrogen concentration in the mother solution of up to 20 ml/L at 25 degrees Celsius or an argon concentration of up to 60ml/L at 25 degrees Celsius.
63. The process for manufacturing according to any of clauses 55 to 62, wherein the step (3) of recovering the radionuclide complex comprises using water-for-injection (WFI) for rinsing.
64. The process for manufacturing according to any of clauses 54 to 63, wherein the step (1) of providing the reaction solution according to clauses 1, 2, 5 to 17, 20 to 23 comprises providing the reaction solution in a container.
65. The process for manufacturing according to any of clauses 54 to 64, wherein the reaction solution has an activity of at least 5 Ci, preferably, of from 5 to 20 Ci, more preferably of 5-15 Ci, even more preferably of 5 - 12, even more preferably of from about 5.4 to 12 Ci, even more preferably from 7 to 12, even more preferably from about 8 to 12 Ci.
66. The process for manufacturing according to any of clauses 54 to 65, wherein the step (2) of reacting the target binding organic molecule with the 177 Lu(III) ions at below atmospheric pressure to obtain the radionuclide complex is carried out over 2 to 15 minutes, preferably 4 to 10 minutes, more preferably 5 min ± 0.5 min.
67. The process for manufacturing according to any of clauses 54 to 66, wherein the step (2) of reacting the target binding organic molecule with the 177 Lu(III) ions at below atmospheric pressure to obtain the radionuclide complex is carried out at 80 to 100 degrees Celsius, preferably 90 to 98 degrees Celsius, more preferably 94 °C ± 4 °C.
68. The process for manufacturing according to any of clauses 54 to 67, wherein a mixture volume at the step (2) of reacting the target binding organic molecule with the 177 Lu(lll) ions at below atmospheric pressure to obtain the radionuclide complex is 15 to 19 ml.
69. The process for manufacturing according to any of clauses 54 to 68, wherein a final volume containing the radionuclide complex after the step (3) of recovering is between 20 to 23 ml.
70. The process for manufacturing according to any of clauses 54 to 69 for manufacturing a radiopharmaceutical solution, further comprising the steps:
   (4) diluting the mother solution with a dilution solution to obtain a dispensing solution at a defined volumetric activity,
   (5) dispensing said dispensing solution into individual patient dose units.
71. The process for manufacturing according to clause 70, wherein the dilution solution comprises:
   (3) a stabilizer against radiolytic degradation, preferably ascorbic acid or salts thereof,
   (4) a sequestering agent, preferably DTPA, and
   (5) optionally an isotonic agent, preferably NaCl.
72. The process for manufacturing according to clause 71, wherein the stabilizer against radiolytic degradation consists of ascorbic acid or salts thereof.
73. The process for manufacturing according to clause 71 or 72, wherein the dilution solution comprises ethanol in not more than 5% (w/w), preferably not more than 2%, even more preferably not more than 1%, even more preferably does not comprise ethanol (substantially 0%).
74. The process for manufacturing according to any of clauses 70 to 73, wherein the defined volumetric activity of the dispensing solution is adjusted to provide individual patient dose units having a volumetric activity of 1000 MBq/mL ± 5% for 177Lu-PSMA-617 and 370 MBq/mL ± 5% for 177Lu-DOTA-TATE.
75. The process for manufacturing according to anyone of clauses 70 to 74, wherein the dispensing solution comprises
   (A) **177Lu-DOTATATE** in an activity in the range from 296 to 444 MBq/mL, preferably 333 to 407 MBq/ml, more preferably about 351 to 389 MBq/ml, most preferably about 370 MBq/mL (10 mCi/mL);
   (B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
   (C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
   (D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
   (E) ascorbic acid or a salt thereof in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
   (F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
   (G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
   (H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.
76. The process for manufacturing according to anyone of clauses 70 to 74, wherein the dispensing solution comprises
   (A) **177Lu-PSMA-617** in an activity in the range from 800 to 1200 MGq/ml, preferably 900 to 1100 MBq/ml, more preferably 950 to 1050 MBq/ml, most preferably about 1000 MBq/mL (27 mCi/mL);
   (B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
   (C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
   (D) gentisic acid or a salt thereof in a concentration in the range from 0.3 to 1.0 mg/mL, preferably 0.3 to 0.5 mg/mL, more preferably 0.31 to 0.47 mg/mL, more preferably 0.35 to 0.43 mg/mL, even more preferably 0.37 to 0.41, most preferably about 0.39 mg/mL with regard to the free acid;
   (E) ascorbic acid or a salt thereof in a concentration in the range from 20 to 52.5, preferably 47.5 to 52.5 mg/mL, more preferably 48 to 52 mg/mL, most preferably about 50 mg/mL with regard to the sodium salt;
   (F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.
77. A Product obtained or obtainable by the process according to anyone of clauses 54 to 74.
78. A product obtained or obtainable by the process according to any of clauses of 70 to 74, wherein the **dispensing solution** comprises:
   (A) **177Lu-DOTATATE** in an activity in the range from 296 to 444 MBq/mL, preferably 333 to 407 MBq/ml, more preferably about 351 to 389 MBq/ml, most preferably about 370 MBq/mL (10 mCi/mL);
   (B) acetic acid in a concentration in the range from 0.384 to 0.576 mg/ml , preferably 0.432 to 0.528 mg/ml, more preferably 0.456 to 0.504 mg/mL, most preferably about 0.48 mg/mL;
   (C) an acetate salt, preferably the sodium salt thereof, in a concentration in the range from 0.528 to 0.792 mg/ml, preferably 0.594 to 0.726 mg/ml , more preferably 0.627 to 0.693 mg/mL, most preferably about 0.66 mg/mL with regard to the sodium salt;
   (D) gentisic acid or a salt thereof in a concentration in the range from 0.504 to 0.756 mg/ml, preferably 0.567 to 0.693 mg/ml, more preferably 0.598 to 0.6615 mg/mL, most preferably about 0.63 mg/mL with regard to the free acid;
   (E) ascorbic acid or a salt thereof in a concentration in the range from 2.24 to 3.36 mg/ml, preferably 2.52 to 3.08 mg/mL, more preferably 2.66 to 2.94 mg/ml, most preferably about 2.8 mg/mL with regard to the sodium salt;
   (F) pentetic acid or a salt thereof in a concentration in the range from 0.04 to 0.06 mg/mL, preferably 0.045 to 0.055 mg/ml, more preferably 0.048 to 0.053 mg/ml, most preferably about 0.050 mg/mL with regard to the free acid;
   (G) sodium chloride in a concentration in the range from 5.55 to 8.15 mg/mL, preferably 6.16 to 7.54 mg/ml, more preferably 6.51 to 7.19 mg/ml, most preferably about 6.85 mg/mL; and
   (H) optionally, sodium hydroxide in a concentration sufficient to provide together with the components (B) and (C) a pH value in the range of 4.5 to 6.0, preferably the sodium hydroxide is present in a concentration in the range from 0.52 to 0.78 mg/ml, preferably 0.59 to 0.72, more preferably 0.618 to 0.683 mg/mL, most preferably 0.65 mg/mL.
79. A product obtained or obtainable by the process according to any of clauses 70 to 74, wherein the **dispensing solution** comprises:
   (A) **177Lu-PSMA-617** in an activity in the range from 800 to 1200 MBq/ml, preferably 900 to 1100 MBq/ml, more preferably 950 to 1050 MBq/ml, most preferably about 1000 MBq/mL (27 mCi/mL);
   (B) acetic acid in a concentration in the range from 0.24 to 0.36 mg/mL, preferably 0.27 to 0.33 mg/mL, more preferably, 0.285 to 0.315 mg/mL, most preferably about 0.3 mg/mL;
   (C) an acetate salt, preferably the sodium salt thereof, in the concentration in the range from 0.33 to 0.49 mg/mL, preferably 0.37 to 0.45 mg/mL, more preferably 0.39 to 0.43 mg/mL, most preferably about 0.41 mg/mL with regard to the sodium salt;
   (D) gentisic acid or a salt thereof in a concentration in the range from 0.3 to 1.0, preferably 0.3 to 0.5, more preferably 0.31 to 0.47 mg/mL, even more preferably 0.35 to 0.43 mg/mL, more preferably 0.37 to 0.41, most preferably about 0.39 mg/mL with regard to the free acid;
   (E) ascorbic acid or a salt thereof in a concentration in the range from 20 to 52.5, preferably 47.5 to 52.5 mg/mL, more preferably 48 to 52 mg/mL, most preferably about 50 mg/mL with regard to the sodium salt;
   (F) pentetic acid or a salt thereof in a concentration in the range from 0.08 to 0.12 mg/mL, preferably 0.09 to 0.11 mg/mL, more preferably 0.095 to 0.105 mg/mL, most preferably about 0.1 mg/mL with regard to the free acid.

## Claims

1. A process for manufacturing a radiopharmaceutical solution comprising the steps:
(1) providing a reaction solution for radiolabelling a target binding organic molecule with ¹⁷⁷Lu(III) ions wherein said reaction solution comprises:
a) ¹⁷⁷Lu(III) ions in a volumetric activity of at least 17 GBq/mL,
b) a target binding organic molecule comprising a target binding organic moiety linked to a chelating moiety suitable for chelating Lu(III) ions, and
c) one or more stabilizers against radiolytic degradation;
(2) reacting the target binding organic molecule comprising the target binding organic moiety linked to the chelating moiety with the ¹⁷⁷Lu(III) ions at below atmospheric pressure to obtain the radionuclide complex in a single container for radiolabeling; and
(3) recovering the radionuclide complex to obtain a mother solution, wherein the recovery of the radionuclide complex comprises introducing or transferring the mother solution in a single container affording a mother solution container, wherein the mother solution is purged with an inert gas before and during introducing or transferring the mother solution in the mother solution container.

2. The process according to claim 1, wherein the reaction solution has an activity of at least 5 Ci, preferably of from 5 to 20 Ci, more preferably of 5 to 15 Ci, even more preferably of 5 to 12 Ci, even more preferably of from about 5.4 to 12 Ci, even more preferably from 7 to 12 Ci, even more preferably from about 8 to 12 Ci.

3. The process according to claim 1 or claim 2, wherein in step (2) the oxygen concentration is lower than 3 mg/L at 25 degrees Celsius.

4. The process according to any one of claims 1 to 3, wherein step (2) is carried out over 2 to 15 minutes, preferably 4 to 10 minutes, more preferably 5 minutes ± 0.5 minutes.

5. The process according to any one of claims 1 to 4, wherein step (2) is carried out at 80 to 100 degrees Celsius, preferably 90 to 98 degrees Celsius.

6. The process according to any one of claims 1 to 5, wherein in step (3) the radionuclide complex is recovered under an inert gas atmosphere.

7. The process according to any one of claims 1 to 6, wherein in step (3) the mother solution container is purged with nitrogen or argon.

8. The process according to claim 7, wherein the mother solution container is purged with nitrogen.

9. The process according to any one of claims 1 to 8, further comprising the steps:
(4) diluting the mother solution with a dilution solution to obtain a dispensing solution at a defined volumetric activity; and
(5) dispensing said dispensing solution into individual patient dose units.

10. The process according to claim 9, wherein the dilution solution comprises:
a) a stabilizer against radiolytic degradation,
b) a sequestering agent, preferably DTPA, and
c) optionally an isotonic agent, preferably NaCl.

11. The process according to claim 10, wherein the stabilizer against radiolytic degradation consists of ascorbic acid or salts thereof.

12. The process according to any one of claims 1 to 11, wherein the target binding organic molecule is a somatostatin receptor binding peptide, optionally wherein the somatostatin receptor binding peptide is linked to a chelating moiety comprising DOTA.

13. The process according to claim 12, wherein the target binding organic molecule is DOTA-TATE.

14. The process according to any one of claims 1 to 11, wherein the target binding organic molecule is a PSMA-binding peptide, optionally wherein the PSMA-binding peptide is linked to a chelating moiety comprising DOTA.

15. The process according to claim 14, wherein the target binding organic molecule is PSMA-617, wherein PSMA-617 has the structure:
